# EUROPEAN PATENT SPECIFICATION

(11) **EP 4 590 256 B1**
(45) Date of publication and mention of the grant of the patent: **04.11.2026**
(21) Application number: 23772263.2
(22) Date of filing: 18.09.2023
(51) Int. Cl.: A61K 8/04, A61K 8/34, A61K 8/41, A61K 8/89, A61Q 5/12

(54) **CONDITIONER WITH IMPROVED MANAGEABILITY**
KONDITIONIERER MIT VERBESSERTER VERWALTBARKEIT
CONDITIONNEUR À GÉRABILITÉ AMÉLIORÉE

(30) Priority: 21.09.2022 EP 22196994
(43) Date of publication of application: 30.07.2025
(73) Proprietor: Wella Germany GmbH, 64295 Darmstadt (DE)
(72) Inventor: SPRINGOB, Christian, 64295 Darmstadt (DE); RUPPACH, Antje, 64295 Darmstadt (DE)
(74) Representative: Ziebig Hengelhaupt Intellectual Property Attorneys Patentanwaltskanzlei PartGmbB
(86) International application number: PCT/EP2023/075619
(87) International publication number: WO 2024/061814

(56) References cited:
- WO-A1-2020/061658
- US-B2- 7 887 787

## Description

### Technical Field

The presently claimed invention is directed to a hair conditioning composition comprising a silicone system comprising a first non-volatile silicone, a second non-volatile silicone and an aminosilicone and a gel matrix comprising a cationic surfactant, a fatty compound and an aqueous carrier and methods for reducing the hair volume by applying the hair conditioning composition.

### Background

Hair consists of many long protein chains composed of amino acid building blocks. These chains, or polymers, are bound to each other via hydrogen bonding, salt bridges between acid and base groups and disulfide bonds. Repeated washing with slightly alkaline shampoos damages the hair by breaking more and more of the disulfide bonds. This causes the cuticle or outer surface of the hair strands to become ruffled and generally leaves the hair in a wet, tangled and generally unmanageable state. This is one cause of "split ends". Once the hair dries, it is often left in a dry, rough, or frizzy condition. Additionally, rough hair catches light unevenly and makes the hair look lustreless and dull. The hair can also be left with increased levels of static upon drying, which can interfere with combing and result in a condition commonly referred to as "fly-away hair".

A higher hair diameter and thicker hair even further increase the unmanageability of hair and associated conditions such as "fly-away hair" and too much hair volume and no volume control, respectively, in particular for long hair.

WO 2012/072765 A1 discloses cosmetic compositions comprising one ore more non-volatile, non-amino polydimethylsiloxanes, one or more acyclic liquid fatty esters of a monoalcohol and one or more amino silicones.

EP 1 713 453 B1 describes a hair conditioning system comprising a mixture of two silicones which are free of amino groups and one aminosilicone. WO2020061658 concerns a hair treatment compositions that provide improved frizz control, style hold, and conditioning to hair. US788778 relates to a hair conditioning composition comprising a silicone system comprising three types of silicones, and a gel matrix, and wherein the composition is prepared by the steps of: the three types of silicones are mixed to form the silicone system; and the silicone system is mixed with the gel matrix.

Therefore, there is a need for hair conditioning compositions that improve the manageability of hair and reduce the unwanted hair volume without compromising other benefits of conventional hair conditioning compositions such as hair shine and hair conditioning. Hence, it is an object of the presently claimed invention to provide hair conditioning compositions that improve the manageability of hair and reduce the unwanted hair volume without compromising other benefits of conventional hair conditioning compositions such as hair shine and hair conditioning.

### Summary

Surprisingly, it was found that the addition of at least one non-volatile silicone with a high viscosity and at least one non-volatile silicone with a low viscosity improves the manageability of hair, reduces the hair volume and reduces the presence of "fly-away hair".

Accordingly, in one aspect, the presently claimed invention is directed to a hair conditioning composition comprising
(A) ≥ 2.0 to ≤ 10 wt.% of a silicone system comprising
   (A1) at least one first non-volatile silicone having a viscosity at 25 °C in the range of ≥ 40,000 to ≤ 90,000 mm2/s, determined according to DIN 053019, which is free of amino groups;
   (A2) at least one second non-volatile silicone having a viscosity at 25 °C in the range of ≥ 100 to ≤ 1,000 mm2/s, determined according to DIN 053019, which is free of amino groups;
   (A3) at least one aminosilicone selected from the group consisting of Quaternium 80 and bisaminopropyl polydimethylsiloxane;
(B) a gel matrix comprising
   (B1) ≥ 0.1 to ≤ 20 wt.% of at least one cationic surfactant selected from the group consisting of quaternary ammonium compounds and amidoamines;
   (B2) ≥ 0.1 to ≤ 20 wt.% of at least one fatty compound having a melting point of ≥ 25 °C; and
(C) at least one aqueous carrier;
wherein each of the wt.% is based on the total weight of the hair conditioning composition.

In another aspect, the presently claimed invention is directed to a method for reducing hair volume comprising the steps of
a) treating the hair with a hair conditioning composition as described herein, and
b) allowing the hair conditioning composition to contact the hair for a period in the range of ≥ 1 to ≤ 60 minutes, before the hair is dried.

In another aspect, the presently claimed invention is directed the use of the hair conditioning composition as described herein for reducing hair volume.

Other objects, advantages and applications of the presently claimed invention will become apparent to those skilled in the art from the following detailed description.

### Detailed description

The following detailed description is merely exemplary in nature and is not intended to limit the presently claimed invention or the application and uses of the presently claimed invention. Furthermore, there is no intention to be bound by any theory presented in the preceding technical field, background, summary, or the following detailed description.

The terms "comprising", "comprises" and "comprised of" as used herein are synonymous with "including", "includes" or "containing", "contains", and are inclusive or open-ended and do not exclude additional, non-recited members, elements, or method steps. It will be appreciated that the terms "comprising", "comprises" and "comprised of" as used herein comprise the terms "consisting of", "consists" and "consists of".

Furthermore, the terms "(a)", "(b)", "(c)", "(d)" etc. and the like in the description and in the claims, are used for distinguishing between similar elements and not necessarily for describing a sequential or chronological order. It is to be understood that the terms so used are interchangeable under appropriate circumstances and that the embodiments of the subject matter described herein are capable of operation in other sequences than described or illustrated herein. In case the terms "(A)", "(B)" and "(C)" or "(a)", "(b)", "(c)", "(d)", "(i)", "(ii)" etc. relate to steps of a method or use or assay there is no time or time interval coherence between the steps, that is, the steps may be carried out simultaneously or there may be time intervals of seconds, minutes, hours, days, weeks, months or even years between such steps, unless otherwise indicated in the application as set forth herein above or below.

In the following passages, different aspects of the subject matter are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to "one embodiment" or "one embodiment" or "preferred embodiment" means that a particular feature, structure, or characteristic described in connection with the embodiment is included in at least one embodiment of the presently claimed invention. Thus, appearances of the phrases "in one embodiment" or "in one embodiment" or "in a preferred embodiment" in various places throughout this specification are not necessarily all referring to the same embodiment but may refer. Furthermore, the features, structures or characteristics may be combined in any suitable manner, as would be apparent to a person skilled in the art from this disclosure, in one or more embodiments. Furthermore, while some embodiments described herein include some, but not other features included in other embodiments, combinations of features of different embodiments are meant to be within the scope of the subject matter, and form different embodiments, as would be understood by those in the art. For example, in the appended claims, any of the claimed embodiments can be used in any combination.

Furthermore, the ranges defined throughout the specification include the end values as well, i.e., a range of 1 to 10 implies that both 1 and 10 are included in the range. For the avoidance of doubt, the applicant shall be entitled to any equivalents according to applicable law.

For the purposes of the presently claimed invention, '% by weight' or 'wt.% 'as used in the presently claimed invention is with respect to the total weight of the coating composition. Further, the sum of wt.-% of all the compounds, as described herein, in the respective components adds up to 100 wt.-%.

Every document cited herein, including any cross referenced or related patent or application, is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests, or discloses any such invention. Further, to the extent that any meaning or definition of a term in this document conflicts with any meaning or definition of the same term in a document incorporated by reference, the meaning or definition assigned to that term in this document shall govern.

The measurement techniques described hereinabove and herein are well known to a person skilled in the art and therefore do not limit the presently claimed invention.

For the purpose of the presently claimed invention, by the expression "conditioning on hair" it is meant imparting positive properties to the hair. For example, "improved conditioning" may cover improved ease of detangling and/or ease of combing. Ease of detangling may be determined by the measurement of the time required for detangling the hair. The shorter the detangling time is, the easier the hair to detangle is. Ease of combing may be determined by the measurement of the work required for combing the hair. The lower the combing work is, the easier the hair to comb is.

For the purpose of the presently claimed invention, by the expression "hair" it is meant keratinous fibre. The term "hair" includes "living" hair, i.e., on a living body, or "non-living", i.e., in a wig, hairpiece or other aggregation of non-living fibers, such as those used in textiles and fabrics. Mammalian hair, e.g., human hair, is preferred in various embodiments. However, animal hair (such as dog or horse), wool, fur and other keratinous fibers are suitable for use in the methods and with the composition described herein.

For the purpose of the presently claimed invention, by the expression "frizz" or "frizzy" hair it is meant that the hair contains short strands sticking up (for example where the hair is parted or elsewhere along the hair length) and projecting away from the main body of the hair; this type of frizz is especially noticeable on people with straight hair who are trying to achieve a smooth style. Additionally, "frizz" may be used to refer to strands of wavy or curly hair that do not align with others to form a defined wave or curl.

For the purpose of the presently claimed invention, by the expression "improved shine" or "enhanced shine" and similar terms relating to shine it is meant that untreated hair, when treated with one or more compounds and subjected to photographic image analysis techniques displays an increase in the reflection of light (e.g., increased gloss and sheen) as compared to identical or similar hair untreated with the same compound or compounds.

For the purposes of the present invention, the term "alkyl" covers acyclic saturated hydrocarbon residues, which may be branched or linear and unsubstituted or at least monosubstituted with, as in the case of C1-C30 alkyl, 1 to 30 (i.e. 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30) C atoms or with, as in the case of C1-C5 alkyl, 1 to 5 (i.e. 1, 2, 3, 4 or 5) C atoms. If one or more of the substituents denote an alkyl residue or comprise an alkyl residue which is mono- or polysubstituted, this is preferably substituted with 1, 2, 3, 4 or 5, particularly preferably with 1, 2 or 3, substituents mutually independently selected from the group consisting of F, Cl, Br, I, -NO₂, -CN, -SH, -NH₂, -N(C₁₋₅-alkyl)₂, -N(C₁₋₅-alkyl)(phenyl), -N(C₁₋₅-alkyl)(CH₂-phenyl), -N(C₁₋₅-alkyl)(CH₂-CH₂-phenyl), -C(=O)-H, - C(=O)-C₁₋₅-alkyl, -C(=O)-phenyl, -C(=S)-C₁₋₅-alkyl, -C(=S)-phenyl, -C(=O)-OH, -C(=O)-O-C₁₋₅-alkyl, -C(=O)-)-phenyl, -C(=O)-NH₂, -C(=O)-NH-C₁₋₅-alkyl, -C(=O)-N(C₁₋₅-alkyl)₂, -S(=O)-C₁₋₅-alkyl, -S(=O)-phenyl, -S(=O)₂-C₁₋₅-alkyl, -S(=O)₂-phenyl, -S(=O)₂-NH₂ and -SO₃H, wherein the above-stated C₁₋₅-alkyl residues are in each case linear or branched and the above-stated phenyl residues are unsubstituted or substituted with 1, 2, 3, 4 or 5 substituents mutually independently selected from the group consisting of F, Cl, Br, I, -CN, -CF₃, -NH₂, -O-CF₃, -SH, -O-CH₃, -O-C₂H₅, -O-C₃H₇, methyl, ethyl, n-propyl, isopropyl, n-butyl, 2-butyl, isobutyl and tert-butyl. Particularly preferred substituents may be selected mutually independently from the group consisting of F, Cl, Br, I, -NO₂, -CN, -SH, -NH₂, -N(CH₃)₂, -N(C₂H₅)₂ and -N(CH₃)(C₂H₅).

In a preferred embodiment, the unsubstituted linear C1-C30 alkyl is preferably selected from the group consisting of methyl, ethyl, propyl, butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, eicosyl, henicosyl, docosyl, tricosyl and tetracosyl; more preferably selected from the group consisting of hexyl, heptyl, octyl, nonyl, decyl, undecyl, dodecyl, tridecyl, tetradecyl, pentadecyl, hexadecyl, heptadecyl, octadecyl, nonadecyl, eicosyl, henicosyl, docosyl, tricosyl and tetracosyl; even more preferably selected from the group consisting of methyl, ethyl, propyl, butyl, pentyl, hexyl, heptyl, octyl, nonyl, decyl, undecyl, dodecyl, tridecyl, tetradecyl and pentadecyl; most preferably selected from the group consisting of methyl, ethyl, propyl, butyl, pentyl, hexyl, heptyl, octyl, nonyl, decyl, undecyl and dodecyl; and in particular preferably selected from the group consisting of methyl, ethyl, propyl, butyl, pentyl, hexyl, heptyl, octyl, nonyl and decyl.

In a preferred embodiment, the unsubstituted branched C1-C30 alkyl is preferably selected from the group consisting of isopropyl, iso-butyl, neo-pentyl, 2-ethyl-hexyl, 2-propyl-heptyl, 2-butyl-octyl, 2-pentyl-nonyl, 2-hexyl-decyl, iso-hexyl, iso-heptyl, iso-octyl, iso-nonyl, iso-decyl, iso-dodecyl, iso-tetradecyl, iso-hexadecyl, iso-octadecyl and iso-eicosyl, more preferably selected from the group consisting of 2-ethyl-hexyl, 2-propyl-heptyl, 2-butyl-octyl, 2-pentyl-nonyl, 2-hexyl-decyl, iso-hexyl, iso-heptyl, iso-octyl, iso-nonyl, iso-decyl, iso-dodecyl, iso-tetradecyl, iso-hexadecyl, iso-octadecyl, iso-eicosyl, 2-methyltricosyl, 2-ethyldocosyl, 3-ethyl-henicosyl, 3-ethylicosyl, 4-propylhenicosyl, propylnonadecyl, 6-butyldodecyl and 5-ethylun-decyl. The polysubstituted alkyl residues are understood to be those alkyl residues which are either poly-, preferably di- or trisubstituted, either on different or on the same C atoms, for example trisubstituted on the same C atom as in the case of -CF₃, or at different locations as in the case of -(CHCl)-(CH₂F). Polysubstitution may proceed with identical or different substituents. Examples which may be mentioned of suitable substituted alkyl residues are -CF₃, -CF₂H, -CFH₂, -(CH₂)-OH, -(CH₂)-NH₂, -(CH₂)-CN, -(CH₂)-(CF₃), -(CH₂)-(CHF₂), -(CH₂)-(CH₂F), -(CH₂)-(CH₂)-O-CH₃, -(CH₂)-(CH₂)-NH₂, -(CH₂)-(CH₂)-CN, -(CF₂)-(CF₃), -(CH₂)-(CH₂)-(CF₃), and -(CH₂)-(CH₂)-(CH₂)-O-CH₃.

In another preferred embodiment, the substituted, linear or branched, C₁-C₃₀ alkyl refers to a branched or linear saturated hydrocarbon group having C1-C30 carbon atoms substituted with functional groups selected from the group consisting of F, Cl, Br, I, -NO₂, -CN, -SH, -NH₂, -N(C₁₋₅-alkyl)₂, -N(C₁₋₅-alkyl)(phenyl), -N(C₁₋₅-alkyl)(CH₂-phenyl), -N(C₁₋₅-alkyl)(CH₂-CH₂-phenyl), -C(=O)-H, -C(=O)-C₁₋₅-alkyl, -C(=O)-phenyl, -C(=S)-C₁₋₅-alkyl, -C(=S)-phenyl, -C(=O)-OH, -C(=O)-O-C₁₋₅-alkyl, -C(=O)-)-phenyl, -C(=O)-NH₂, -C(=O)-NH-C₁₋₅-alkyl, -C(=O)-N(C₁₋₅-alkyl)₂, -S(=O)-C₁₋₅-alkyl, -S(=O)-phenyl, -S(=O)₂-C₁₋₅-alkyl, -S(=O)₂-phenyl, -S(=O)₂-NH₂ and -SO₃H, wherein the above-stated C₁₋₅-alkyl residues are in each case linear or branched and the above-stated phenyl residues are preferably unsubstituted or substituted with 1, 2, 3, 4 or 5 substituents mutually independently selected from the group consisting of F, Cl, Br, I, -CN, - CF₃, -NH₂, -O-CF₃, -SH, -O-CH₃, -O-C₂H₅, -O-C₃H₇, methyl, ethyl, n-propyl, isopropyl, n-butyl, 2-butyl, isobutyl and tert-butyl. Particularly preferred substituents may be selected mutually independently from the group consisting of F, Cl, Br, I, -NO₂, -CN, -SH, -NH₂, -N(CH₃)₂, - N(C₂H₅)₂ and -N(CH₃)(C₂H₅).

In another preferred embodiment, the substituted, linear or branched, C₁-C₃₀ alkyl refers to a branched or linear saturated hydrocarbon group having C1-C30 carbon atoms substituted with functional groups selected from the group consisting of alkoxy, C(=O)R, CN and SR, preferably selected from the group consisting of 1-methoxy methyl, 1-methoxy methyl, 1-methoxy ethyl, 1-methoxy propyl, 1-methoxy butyl, 1-methoxy pentyl, 1-methoxy hexyl, 1-methoxy heptyl, 1-methoxy octyl, 1-methoxy nonyl, decyl, 1-methoxy undecyl, 1-methoxy dodecyl, 1-methoxy tridecyl, 1-methoxy tetradecyl, 1-methoxy pentadecyl, 1-methoxy hexadecyl, 1-methoxy heptadecyl, 1-methoxy octadecyl, 1-methoxy nonadecyl, 1-methoxy eicosyl, 1-methoxy henicosyl, 1-methoxy docosyl, 1-methoxy tricosyl, 1-methoxy tetracosyl, 2-methoxy propyl, 2-methoxy butyl, 2-methoxy pentyl, 2-methoxy hexyl, 2-methoxy heptyl, 2-methoxy octyl, 2-methoxy nonyl, decyl, 2-methoxy undecyl, 2-methoxy dodecyl, 2-methoxy tridecyl, 2-methoxy tetradecyl, 2-methoxy pentadecyl, 2-methoxy hexadecyl, 2-methoxy heptadecyl, 2-methoxy octadecyl, 2-methoxy nonadecyl, 2-methoxy eicosyl, 2-methoxy henicosyl, 2-methoxy docosyl, 2-methoxy tricosyl, 2-methoxy tetracosyl, 1-acetoxy methyl, 1-acetoxy ethyl, 1-acetoxy propyl, 1-acetoxy butyl, 1-acetoxy pentyl, 1-acetoxy hexyl, 1-acetoxy heptyl, 1-acetoxy octyl, 1-acetoxy nonyl, decyl, 1-acetoxy undecyl, 1-acetoxy dodecyl, 1-acetoxy tridecyl, 1-acetoxy tetradecyl, 1-acetoxy pentadecyl, 1-acetoxy hexadecyl, 1-acetoxy heptadecyl, 1-acetoxy octadecyl, 1-acetoxy nonadecyl, 1-acetoxy eicosyl, 1-acetoxy henicosyl, 1-acetoxy docosyl, 1-acetoxy tricosyl, 1-acetoxy tetracosyl, 1-cyano methyl, 1-cyano ethyl, 1-cyano propyl, 1-cyano butyl, 1-cyano pentyl, 1-cyano hexyl, 1-cyano heptyl, 1-cyano octyl, 1-cyano nonyl, decyl, 1-cyano undecyl, 1-cyano dodecyl, 1-cyano tridecyl, 1-cyano tetradecyl, 1-cyano pentadecyl, 1-cyano hexadecyl, 1-cyano heptadecyl, 1-cyano octadecyl, 1-cyano nonadecyl, 1-cyano eicosyl, 1-cyano henicosyl, 1-cyano docosyl, 1-cyano tricosyl, 1-cyano tetracosyl, 2-cyano propyl, 2-cyano butyl, 2-cyano pentyl, 2-cyano hexyl, 2-cyano heptyl, 2-cyano octyl, 2-cyano nonyl, decyl, 2-cyano undecyl, 2-cyano dodecyl, 2-cyano tridecyl, 2-cyano tetradecyl, 2-cyano pentadecyl, 2-cyano hexadecyl, 2-cyano heptadecyl, 2-cyano octadecyl, 2-cyano nonadecyl, 2-cyano eicosyl, 2-cyano henicosyl, 2-cyano docosyl, 2-cyano tricosyl, 2-cyano tetracosyl, 1-thioyl methyl, 1-thioyl ethyl, 1-thioyl propyl, 1-thioyl butyl, 1-thioyl pentyl, 1-thioyl hexyl, 1-thioyl heptyl, 1-thioyl octyl, 1-thioyl nonyl, decyl, 1-thioyl undecyl, 1-thioyl dodecyl, 1-thioyl tridecyl, 1-thioyl tetradecyl, 1-thioyl pentadecyl, 1-thioyl hexadecyl, 1-thioyl heptadecyl, 1-thioyl octadecyl, 1-thioyl nonadecyl, 1-thioyl eicosyl, 1-thioyl henicosyl, 1-thioyl docosyl, 1-thioyl tricosyl and 1-thioyl tetracosyl.

For the purposes of the present invention, the term alkenyl denotes unsubstituted, linear C₂-C₃₀ alkenyl which is preferably selected from the group consisting of 1-propenyl, 1-butenyl, 1-pentenyl, 1-hexenyl, 2-hexenyl, 1-heptenyl, 2-heptenyl, 1-octenyl, 2-octenyl, 1-nonenyl, 2-nonenyl, 1-decenyl, 2-decenyl, 1-undecenyl, 2-undecenyl, 1-dodecenyl, 2-dodecenyl, 1-tridecenyl, 2-tridecenyl, 1-tetradecenyl, 2-tetradecenyl, 1-pentadecenyl, 2-pentadecenyl, 1-hexadecenyl, 2-hexadecenyl, 1-heptadecenyl, 2-heptadecenyl, 1-octadecenyl, 2-octadecenyl, 1-nonadecenyl, 2-nonadecenyl, 1-eicosenyl and 2-eicosenyl, more preferably selected from 1-hexenyl, 2-hexenyl, 1-heptenyl, 2-heptenyl, 1-octenyl, 2-octenyl, 1-nonenyl, 2-nonenyl, 1-decenyl, 2-decenyl, 1-undecenyl, 2-undecenyl, 1-dodecenyl, 2-dodecenyl, 1-tridecenyl, 2-tridecenyl, 1-tetradecenyl, 2-tetradecenyl, 1-pentadecenyl, 2-pentadecenyl, 1-hexadecenyl, 2-hexadecenyl, 1-heptadecenyl, 2-heptadecenyl, 1-octadecenyl, 2-octadecenyl, 1-nonadecenyl, 2-nonadecenyl, 1-eicosenyl and 2-eicosenyl, 20-henicosenyl, 2-docosenyl, 6-tricosenyl and 2-tetracosenyl.

In a preferred embodiment, the unsubstituted branched C₂-C₃₀ alkenyl is selected from the group consisting of isopropenyl, iso-butenyl, neo-pentenyl, 2-ethyl-hexenyl, 2-propyl-heptenyl, 2-butyl-octenyl, 2-pentyl-nonenyl, 2-hexyl-decenyl, iso-hexenyl, iso-heptenyl, iso-octenyl, iso-nonenyl, iso-decenyl, iso-dodecenyl, iso-tetradecenyl, iso-hexadecenyl, iso-octadecenyl, iso-eicosenyl, 2-methyl tricosenyl, 2-ethyl docosenyl, 3-ethylhenicosenyl, 3-ethyl icosenyl, 4-propylhenicosenyl, 4-propylnonadecenyl, 6-butyldodecenyl, 5-ethylundedcenyl, 1,4-hexadienyl, 1,3-hexadienyl, 2,5-hexadienyl, 3,5-hexadienyl, 2,4-hexadienyl, 1,3,5-hexa-trienyl, 1,3,6-heptatrienyl, 1,4,7-octatrienyl or 2-methyl-1,3,5hexatrienyl, 1,3,5,7-octate-traenyl, 1,3,5,8-nonatetraenyl, 1,4,7,10-undecatetraenyl, 2-ethyl-1 ,3,6,8-nonatetraenyl, 2-ethenyl-1,3,5,8-nonatetraenyl, 1,3,5,7,9-decapentaenyl, 1,4,6,8,10-undecapentaenyl, and 1,4,6,9,11-dodecapentaenyl.

For the purposes of the present invention, the substituted, linear or branched, C2-C30 alkenyl refers to a branched or an linear unsaturated hydrocarbon group having C2-C30 carbon atoms substituted with functional groups selected from alkoxy, C(=O)R, CN and SR; wherein R is hydrogen, substituted or unsubstituted, linear or branched C1-C30 alkyl, substituted or unsubstituted, linear or branched C2-C30 alkenyl, substituted or unsubstituted C5-C30 cycloalkyl and substituted or unsubstituted C6-C30 aryl.

For the purposes of the present invention, the term alkenyl refers to a branched or an linear unsaturated hydrocarbon group having C₂-C₃₀ carbon atoms substituted with functional groups selected from, alkoxy, C(=O)R, CN and SR; preferably selected from the group consisting of 1-methoxy ethenyl, 2-methoxy propenyl, 4-methoxy butenyl, 3-methoxy pentenyl, 5-methoxy hexenyl, 2-methoxy heptenyl, 5-methoxy octenyl, 3-methoxy nonenyl, 6-methoxy undecenyl, 1-methoxy dodec-2-enyl, 1-methoxy tridec-5-enyl, 3-methoxy tetradic-5-enyl, 3-methoxy pentade-12-encyl, 10-methoxy hexadec-15-enyl, 12-methoxy heptadic-16-enyl,1-methoxy octadec-3-enyl, 1-methoxy nonadec-2-enyl, 1-methoxy eicos-20-enyl, 1-methoxy henicos-2-enyl, 1-methoxy docos-4-enyl, 1-methoxy tricos-22-enyl, 1-methoxy tetracos-23-enyl, 2-methoxy prop-1-enyl, 2-methoxy but-1-enyl, 2-methoxy pent-4-enyl, 2-methoxy hex-2-enyl, 2-methoxy hept-3-enyl, 2-methoxy oct-7-enyl, 2-methoxy non-5-enyl, 2-methoxy undec-10-enyl, 2-methoxy dodec-4-enyl, 2-methoxy tridec-12-enyl, 2-methoxy tetradic-10-enyl, 2-methoxy pentadec-14-enyl, 2-methoxy hexadec-1-enyl, 2-methoxy heptadic-1-enyl, 2-methoxy octadic-12-enyl, 2-methoxy nonadec-10-enyl, 2-methoxy eicos-18-enyl, 2-methoxy henicos-2-enyl, 2-methoxy docos-3-enyl, 20-methoxy tricos-2-enyl, 21-methoxy tetracos-4-enyl, 1-acetoxy ethenyl, 1-acetoxy prop-1-enyl, 1-acetoxy but-2-enyl, 1-acetoxy pent-4-enyl, 1-acetoxy hex-2-enyl, 1-acetoxy hept-1-enyl, 1-acetoxy oct-7-enyl, 1-acetoxy non-2-enyl, 5-acetoxy dec-3-enyl, 1-acetoxy undec-10-enyl, 1-acetoxy dodec-2-enyl, 1-acetoxy tridec-12-enyl, 10-acetoxy tetradec-2-enyl, 15-acetoxy pentadec-2-enyl, 10-acetoxy hexadec-2-enyl, 11-acetoxy heptadec-1-enyl, 13-acetoxy octadec-2-enyl, 1-acetoxy nonadec-14-enyl, 20-acetoxy eicos-19-enyl, 1-acetoxy henicos-2-enyl, 1-acetoxy docos-10-enyl, 1-acetoxy tricos-22-enyl, 1-acetoxy tetracos-23-enyl, 1-cyano eth-1-enyl, 1-cyano prop-2-enyl, 1-cyano but-2-enyl, 1-cyano pent-3-enyl, 1-cyano hex-5-enyl, 1-cyano hept-6-enyl, 1-cyano oct-2-enyl, 1-cyano non-3-enyl, 11-cyano undec-2-enyl, 10-cyano dodec-2-enyl, 10-cyano tridec-12-enyl, 1-cyano tetradec-3-enyl, 1-cyano pentadec-14-enyl, 1-cyano hexadec-15-enyl, 1-cyano heptadec-2-enyl, 1-cyano octadec-3-enyl, 1-cyano nonadec-18-enyl, 1-cyano eicos-10-enyl, 1-cyano henicos-20-enyl, 15-cyano docos-3-enyl, 1-cyano tricos-20-enyl, 1-cyano tetracos-2-enyl, 2-cyano prop-2-enyl, 2-cyano but-1-enyl, 2-cyano pent-1-enyl, 2-cyano hex-3-enyl, 2-cyano hept-6-enyl, 2-cyano oct-1-enyl, 2-cyano non-8-enyl, 2-cyano undec-10-enyl, 2-cyano dodec-1-enyl, 2-cyano tridec-12-enyl, 2-cyano tetradec-10-enyl, 2-cyano pentadec-3-enyl, 2-cyano hexadec-2-enyl, 2-cyano heptadec-1-enyl, 2-cyano octadec-12-enyl, 2-cyano nonadec-15-enyl, 2-cyano eicos-1-enyl, 2-cyano henicos-5-enyl, 2-cyano docos-20-enyl, 2-cyano tricos-22-enyl, 2-cyano tetracos-20-enyl, 1-thionyl eth-1-enyl, 1-thionyl prop-2-enyl, 1-thionyl but-2-enyl, 1-thionyl pent-4-enyl, 1-thionyl hex-2-enyl, 1-thionyl hept-5-enyl, 1-thionyl oct-3-enyl, 1-thionyl non-5-enyl, 1-thionyl undec-10-enyl, 1-thionyl dodec-11-enyl, 1-thionyl tridec-2-enyl, 1-thionyl tetradec-4-enyl, 1-thionyl pentadec-5-enyl, 1-thionyl hexadec-3-enyl, 1-thionyl heptadec-2-enyl, 1-thionyl octadec-3-enyl, 1-thionyl nonadec-15-enyl, 1-thionyl eicos-18-enyl, 1-thionyl henicos-20-enyl, 1-thionyl docos-21-enyl, 1-thionyl tricos-20-enyl and 1-thionyl tetracos-22-enyl.

For the purposes of the present invention, the term "heteroalkyl" refers to an alkyl group, in which one or more carbon atoms have in each case been replaced by a heteroatom mutually independently selected from the group consisting of oxygen, sulfur and nitrogen (NH). Heteroalkyl residues preferably comprise 1, 2 or 3 heteroatom(s) mutually independently selected from the group consisting of oxygen, sulfur and nitrogen (NH) as chain link(s). Heteroalkyl residues may preferably be 2- to 12-membered, particularly preferably 2- to 6-membered.

For the purposes of the present invention, the term "heteroalkenyl" refers to an alkenyl group, in which one or more carbon atoms have in each case been replaced by a heteroatom mutually independently selected from the group consisting of oxygen, sulfur and nitrogen (NH). Heteroalkenyl residues preferably comprise 1, 2 or 3 heteroatom(s) mutually independently selected from the group consisting of oxygen, sulfur and nitrogen (NH) as chain link(s). Heteroalkenyl residues may preferably be 3- to 12-membered, particularly preferably 3- to 6-membered.

For the purposes of the present invention, the term "cycloalkyl" refers to a monocyclic and bicyclic saturated cycloaliphatic radical having 5 to 30 carbon atoms. Representative examples of unsubstituted or branched C5-C30 monocyclic and bicyclic cycloalkyl include, but are not limited to, cyclopentyl, cyclohexyl, cycloheptyl, cyclooctyl, bicyclo[2.2.1]heptyl, and bicyclo[3.1.1]heptyl.

In another preferred embodiment, the C5-C30 monocyclic and bicyclic cycloalkyl can be branched with one or more equal or different alkyl groups such as methyl, ethyl, n-propyl, iso-propyl, n-butyl, iso-butyl, n-pentyl, iso-pentyl, neo-pentyl etc. The representative examples of branched C3-C10 monocyclic and bicyclic cycloalkyl include, but are not limited to, methyl cyclohexyl and dimethyl cyclohexyl.

For the purposes of the present invention, the term "aryl" refers to an aromatic compound that may have more than one aromatic ring. The representative examples for substituted and unsubstituted C6-C30 aryl include phenyl, naphthyl, anthracenyl, tetraphenyl, phenalenyl and phenanthrenyl.

For the purposes of the present invention, the term "alkylene" covers acyclic saturated hydrocarbon residues, which may be acyclic saturated hydrocarbon chains, which combine different moieties, as in the case of C1-C30 alkylene with 1 to 30 (i.e. 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30) C atoms or with, as in the case of C1-C10 alkylene, 1 to 10 (i.e. 1, 2, 3, 4, 5, 6, 7, 8, 9 or 10) C atoms. Representative examples of the alkylene groups include, but are not limited to, -CH₂-CH₂-CH₂-CH(CH₃)-, -CH₂-CH(CH₂CH₃)-, -CH₂-CH(n-C₃H₇)-, -CH₂-CH(n-C₄H₉)-, -CH₂-CH(n-C₅H₁₁)-, -CH₂-CH(n-C₆H₁₃)-, -CH₂-CH(n-C₇H₄₅)-, -CH₂-CH(n-C₈H₁₇)-, -CH(CH₃)-CH(CH₃)-, -C(CH₃)₂-, -CH₂-C(CH₃)₂-CH₂-, and -CH₂-[C(CH₃)₂]₂-CH₂-, -(CH₂)₃-, -(CH₂)₄-, -(CH₂)₅-, - (CH₂)₆-, -(CH₂)₈-, -(CH₂)₁₀-, -(CH₂)₇-, -(CH₂)₉-, -(CH₂)₁₁-, -(CH₂)₁₂-, -(CH₂)₁₃-, -(CH₂)₁₄-, - (CH₂)₁₅-, -(CH₂)₁₆-, -(CH₂)₁₇-, -(CH₂)₁₈-, -(CH₂)₁₉-, -(CH₂)₂₀-, -(CH₂)₂₁-, -(CH₂)₂₂-, -(CH₂)₂₃-, - (CH₂)₂₄-, -(CH₂)₂₅-, -(CH₂)₂₆-, -(CH₂)₂₇-, -(CH₂)₂₈-, -(CH₂)₂₉- and -(CH₂)₃₀-.

The below materials are being used in the embodiments of the presently claimed invention. The presently claimed invention is described with non-limiting embodiments. The listed ingredients are the preferred features as well as other preferred but non-limiting features of the presently claimed invention.

An aspect of the presently claimed invention is directed to a hair conditioning composition comprising
(A) ≥ 2.0 to ≤ 10 wt.% of a silicone system comprising
   (A1) at least one first non-volatile silicone having a viscosity at 25 °C in the range of ≥ 40,000 to ≤ 90,000 mm2/s, determined according to DIN 053019, which is free of amino groups;
   (A2) at least one second non-volatile silicone having a viscosity at 25 °C in the range of ≥ 100 to ≤ 1,000 mm2/s, determined according to DIN 053019, which is free of amino groups;
   (A3) at least one aminosilicone selected from the group consisting of Quaternium 80 and bisaminopropyl polydimethylsiloxane;
(B) a gel matrix comprising
   (B1) ≥ 0.1 to ≤ 20 wt.% of at least one cationic surfactant selected from the group consisting of quaternary ammonium compounds and amidoamines;
   (B2) ≥ 0.1 to ≤ 20 wt.% of at least one fatty compound having a melting point of ≥ 25°C; and
(C) at least one aqueous carrier;
wherein each of the wt.% is based on the total weight of the hair conditioning composition.

Quaternium 80 in the present case means siloxanes and silicones, di-Me, 3-[3-[(3-coco amidopropyl)dimethylammonio]-2-hydroxypropoxy]propyl group-terminated, acetates and salts thereof. Quaternium 80 has the CAS-number 134737-05-6.

In one embodiment, the at least one first non-volatile silicone (A1) and at least one first non-volatile silicone (A2) are preferably selected from the following polydimethylsiloxanes types:
S1: pure polydimethylsiloxanes, i.e., linear or Si-branched polysiloxane which essentially consist of dimethylsilyloxy groups and terminal trimethylsilyloxy groups, preferably those with M_{w} in the range from ≥ 100,000 to ≤ 130,000 g/mol as in the case of compound (A1) and those with M_{w} in the range from ≥ 5,000 to ≤ 30,000 g/mol as in the case of compound (A2);
S2: α,ω- terminated polydimethylsiloxanes, i.e., linear or Si-branched polysiloxanes having terminal C2-C30-alkyl groups, which are interrupted by at least one functional moiety selected from -O-, -O-C(=O)- or terminal C₁-C₁₀-alkoxy groups,
   preferably those with M_{w} in the range from ≥ 100,000 to ≤ 130,000 g/mol as in the case of compound (A1) and those with M_{w} in the range from ≥ 5,000 to ≤ 30,000 g/mol as in the case of compound (A2);
S3: Polysiloxanes of the formula

   R-Si(CH₃)₂-O-[Si(CH₃)₂-O]ₚ-[Si(R^{a})(CH₃)-O]_{q}Si(CH₃)₂R;

   where q is an integer from 1 to 500 and p is and an integer from 0 to 500 with p + q being at least 2, in particular at least 5, e.g., from 2 to 1000, in particular from 5 to 500;
   - R: is CH₃, C2-C22 alkyl, halogenated C1-C22 alkyl, C5-C22 cycloalkyl, or an aromatic hydrocarbon radical having 6 to 22 carbon atoms, such as phenyl or phenyl-C1-C4-alkyl, and
   - R^{a}: is C2-C22 alkyl, halogenated C1-C22 alkyl, C5-C22 cycloalkyl, aryl having 6 to 22 carbons, such as phenyl or phenyl-C1-C4-alkyl, or -[Si(CH₃)₂-O]ₙ-[Si(R^{b})(CH₃)-O]ₘSi(CH₃)₂R,
   where n and m are identical or different and an integer from 1 to 100 with n + m being at least 1,
   R is as defined above and
   R^{b} is a radical selected from C2-C22 alkyl, halogenated C1-C22 alkyl, C5-C22 cycloalkyl and aryl having 6 to 22 carbons, such as phenyl or phenyl-C1-C4-alkyl.

In the polysiloxanes S3, the moieties -[Si(CH₃)₂-O]- and -[Si(R^{a})(CH₃)-O]- and, if present, moieties -[Si(CH₃)₂-O]- and -[Si(R^{b})(CH₃)-O]- may be arranged statistically or blockwise. The polysiloxanes S3 preferably have a M_{w} in the range from ≥ 100,000 to ≤ 300,000 g/mol as in the case of compound (A1) and a M_{w} in the range from ≥ 5,000 to ≤ 30,000 g/mol as in the case of compound (A2).

### S4: Polysiloxanes of the formula

R'-Si(CH₃)₂-O-[Si(CH₃)₂-O]ⱼ-[Si(R^{c})(CH₃)-O]ₖSi(CH₃)₂R';

where k is an integer from 1 to 500 and j is and an integer from 0 to 500 with j + k being at least 2, in particular at least 5, e.g., from 2 to 1000, in particular from 5 to 500;
- R': is CH₃, halogenated C1-C22 alkyl, or a C2-C30-alkyl group, which is interrupted by at least one functional moiety selected from -O- or -O-C(=O)-, and
- R^{c}: is a C2-C30-alkyl group, which is interrupted by at least one functional moiety selected from -O- or -O-C(=O)-, or -[Si(CH₃)₂-O]ₙ-[Si(R^{d})(CH₃)-O]ₘSi(CH₃)₂R',
where n and m are identical or different and an integer from 1 to 100 with n + m being at least 1,
- R': is as defined above and
- R^{d}: is a C2-C30-alkyl group, which is interrupted by at least one functional moiety selected from -O- or -O-C(=O)-.

In the polysiloxanes S4, the moieties -[Si(CH₃)₂-O]- and -[Si(R^{c})(CH₃)-O]- and, if present, moieties -[Si(CH₃)₂-O]- and -[Si(R^{d})(CH₃)-O]- may be arranged statistically or blockwise. The polysiloxanes S4 preferably have a M_{w} in the range from ≥ 100,000 to ≤ 300,000 g/mol as in the case of compound (A1) and a M_{w} in the range from ≥ 5,000 to ≤ 30,000 g/mol as in the case of compound (A2).

In one embodiment, the at least one first non-volatile silicone (A1) is selected from the group consisting of polydimethylsiloxanes, polydiethylsiloxanes and polymethylphenylsiloxanes; more preferably the at least one first non-volatile silicone (A1) is polydimethylsiloxane. In one embodiment, the at least one first non-volatile silicone (A2) is selected from the group consisting of polydimethylsiloxanes, polydiethylsiloxanes and polymethylphenylsiloxanes; more preferably the at least one first non-volatile silicone (A2) is polydimethylsiloxane.

For the purposes of the presently claimed invention, the term "non-volatile" refers to a silicone which at ambient temperature, i.e., 25 °C, is capable of remaining on the hair for several hours. A non-volatile silicone has preferably a vapour pressure at ambient temperature, i.e., 25 °C, of ≤ 2.66 Pa, more preferably ≤ 0.13 Pa-
In one embodiment, the at least one first non-volatile silicone (A1) and the at least one second non-volatile silicone (A2) do not contain any amino functionality and are different from the at least one aminosilicone (A3).

In one embodiment, the at least one first non-volatile silicone (A1) and the at least one second non-volatile silicone (A2) are preferably polydimethylsiloxanes which are insoluble in the hair conditioning composition of the presently claimed invention and are present in the form of oils, waxes, resins or gums.

In another preferred embodiment of the presently claimed invention, the at least one first non-volatile silicone (A1) is a liquid at 100°C, more preferably at 40°C. In another preferred embodiment of the presently claimed invention, the at least one first non-volatile silicone (A2) is a liquid at 100°C, more preferably at 40°C.

In one embodiment, the silicone system (A) is preferably present in an amount in the range of ≥ 2.5 to ≤ 8.0 wt.%, more preferably in the range of ≥ 2.7 to ≤ 5.0 wt.%, even more preferably in the range of ≥ 3.0 to ≤ 5.0 wt.%, based on the total weight of the hair conditioning composition.

In one embodiment, the at least one component (A1) preferably has a viscosity at 25 °C in the range of ≥ 45,000 to ≤ 85,000 mm2/s, more preferably in the range of ≥ 50,000 to ≤ 80,000 mm2/s, even more preferably in the range of ≥ 55,000 to ≤ 70,000 mm2/s, determined according to DIN 053019.

In one embodiment, the at least one component (A1) is preferably present in an amount in the range of ≥ 1.0 to ≤ 5.0 wt.%, more preferably in the range of ≥ 1.5 to ≤ 4.0 wt.%, even more preferably in the range of ≥ 1.5 to ≤ 3.0 wt.%, most preferably in the range of ≥ 1.5 to ≤ 2.5 wt.%, based on the total weight of the hair conditioning composition.

In one embodiment, the at least one component (A2) preferably has a viscosity at 25 °C in the range of ≥ 200 to ≤ 900 mm2/s, more preferably in the range of ≥ 250 to ≤ 800 mm2/s, even more preferably in the range of ≥ 300 to ≤ 700 mm2/s, most preferably in the range of ≥ 400 to ≤ 600 mm2/s, determined according to DIN 053019.

In one embodiment, the at least one component (A2) is preferably present in an amount in the range of ≥ 1.0 to ≤ 5.0 wt.%, more preferably in the range of ≥ 1.5 to ≤ 4.0 wt.%, even more preferably in the range of ≥ 1.5 to ≤ 3.0 wt.%, most preferably in the range of ≥ 1.5 to ≤ 2.5 wt.%, based on the total weight of the hair conditioning composition.

In one embodiment, the weight ratio of the at least one component (A1) to the at least one component (A2) is preferably in the range of ≥ 0.5:1.0 to ≤ 2.0:1.0, more preferably in the range of ≥ 0.8:1.0 to ≤ 1.30:1.0.

In one embodiment, the at least one aminosilicone (A3) denotes any silicone comprising at least one primary, secondary or tertiary amine or quaternary ammonium. In one embodiment, the at least one aminosilicone (A3) is selected from the group consisting of amine-functionalized silicones (e.g., amodimethicone), Quaternium 80, bisaminopropyl polydimethylsiloxanes (bisaminopropyl dimethicone) and trimethyl silylamodimethicone; more preferably the at least one component (A3) is selected from the group consisting of Quaternium 80 and bisaminopropyl polydimethylsiloxane.

In one embodiment, the at least one aminosilicone (A3) is different from the at least one cationic surfactant (B1).

In one embodiment, the at least one aminosilicone (A3) has a weight average molecular weight M_{w} in the range from ≥ 2,000 to ≤ 50,000 g/mol; more preferably in the range from ≥ 3,000 to ≤ 40,000 g/mol.

In one embodiment, the at least one component (A3) is preferably present in an amount in the range of ≥ 0.5 to ≤ 6.0 wt.%, more preferably in the range of ≥ 1.0 to ≤ 3.0 wt.%, even more preferably in the range of ≥ 1.2 to ≤ 2.5 wt.%, most preferably in the range of ≥ 1.2 to ≤ 2.0 wt.%, based on the total weight of the hair conditioning composition.

In a preferred embodiment, the weight ratio of the total amount of the at least one component (A1) and the at least one component (A2) to the at least one component (A3) is preferably in the range of ≥ 4.0:1.0 to ≤ 1.0:1.0, more preferably in the range of ≥ 2.0:1.0 to ≤ 2.0:1.0.

The hair conditioning composition of the presently claimed invention comprises a gel matrix comprising at least one cationic surfactant (B1), at least one fatty compound having a melting ≥ 25 °C and an aqueous carrier.

In one embodiment, the at least one cationic surfactant can be a cationic surfactant system comprising mono-long alkyl quaternized ammonium salts as encompassed by formula (I), a combination of mono-long alkyl quaternized ammonium salts as encompassed by formula (I) and di-long alkyl quaternized ammonium salts as encompassed by formula (I), mono-long alkyl amidoamine salt as encompassed by formula (II), a combination of a mono-long alkyl amidoamine salt as encompassed by formula (II) and a di-long alkyl quaternized ammonium salt as encompassed by formula (I), a combination of a mono-long alkyl amidoamine salt as encompassed by formula (II) and a mono-long alkyl quaternized ammonium salt as encompassed by formula (I).

**In** one embodiment, the at least one cationic surfactant (B1) is substantially free of silicone.

**In** one embodiment, the at least one cationic surfactant (B1) is preferably selected from the group consisting of quaternary ammonium compounds and amidoamines.

**In** one embodiment, the quaternary ammonium compounds preferably have the general formula (I), 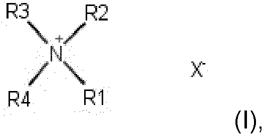 wherein
- R1: is selected from the group consisting of linear or branched, substituted or unsubstituted C1-C30 alkyl, linear or branched, substituted or unsubstituted C2-C30 alkenyl, substituted or unsubstituted, linear or branched C1-C30 heteroalkyl and substituted or unsubstituted, linear or branched C2-C30 heteroalkenyl;
- R2: is selected from the group consisting of linear or branched, substituted or unsubstituted C6-C30 alkyl, linear or branched, substituted or unsubstituted C6-C30 alkenyl, substituted or unsubstituted, linear or branched C6-C30 heteroalkyl and substituted or unsubstituted, linear or branched C6-C30 heteroalkenyl;
- R3 and R4: are, identical or different, are selected from the group consisting of linear or branched, substituted or unsubstituted C1-C30 alkyl, linear or branched, substituted or unsubstituted C2-C30 alkenyl, substituted or unsubstituted C6-C30 aryl and substituted or unsubstituted C1-C10 alkylene C6-C30 aryl; and
- X⁻: is selected from the group consisting of halide, alkyl sulfates, phosphates, alkyl sulfonates, alkylaryl sulfonates and anions derived from organic acids and amino acids;

In a preferred embodiment, R1 is linear or branched, unsubstituted C1-C30 alkyl;
- R2: is linear or branched, unsubstituted C6-C30 alkyl;
- R3 and R4: are, identical or different, linear or branched, unsubstituted C1-C4 alkyl; and
- X⁻: is halide; more preferably the quaternary ammonium compounds are selected from the group consisting of cetrimonium halide, stearimonium halide, behentrimonium halide, dodecyltrimethylammonium halide, didodecyldimethylammonium halide, tetradecyltrimethylammonium halide, distearyldimethylammonium halide and dicetyldimethylammonium halide.

In one embodiment, the amidoamines preferably have the general formula (II),

R5-C(=O)-NH-(CH₂)n-NR6R7 (II),

wherein
- R5: is selected from the group consisting of linear or branched, substituted or unsubstituted C1-C30 alkyl, linear or branched, substituted or unsubstituted C2-C30 alkenyl,
- R3 and R4: are, identical or different, are selected from the group consisting of linear or branched, substituted or unsubstituted C6-C30 alkyl, linear or branched, substituted or unsubstituted C6-C30 alkenyl, substituted or unsubstituted, linear or branched C6-C30 heteroalkyl, substituted or unsubstituted, linear or branched C6-C30 heteroalkenyl, substituted or unsubstituted C6-C30 aryl and substituted or unsubstituted C1-C10 alkylene C6-C30 aryl; and
- n: is 1, 2, 3 or 4.

In one embodiment, the amidoamine is preferably selected from the group consisting of behenamidopropyldimethylamine, behenamidopropyldiethylamine, behenamidoethyldiethylamine, behenamidoethyldimethylamine, arachidamidopropyldimethylamine, arachidamidopropyldiethylamine, arachidamidoethyidiethylamine and arachidamidoethyidimethylamine.

In a preferred embodiment, the at least one component (B1) is different from the at least one component (A3).

**In** a one embodiment, the at least one component (B1) is preferably present in an amount in the range of ≥ 1.0 to ≤ 4.0 wt.%, more preferably in the range of ≥ 2.0 to ≤ 3.0 wt.%, based on the total weight of the hair conditioning composition.

**In** one embodiment, the at least one component (B2) is preferably selected from the group consisting of fatty alcohols, fatty acids, fatty alcohol derivatives and fatty acid derivatives.

**In** one embodiment, the fatty alcohols preferably have from 14 to 30 carbon atoms, more preferably from 16 to 22 carbon atoms and can be linear or branched; more preferably the fatty alcohols are selected from the group consisting of cetyl alcohol, stearyl alcohol and behenyl alcohol.

**In** one embodiment, the fatty acids preferably have from 10 to 30 carbon atoms, more preferably from 12 to 22 carbon atoms, and more preferably from 16 to 22 carbon atoms and can be linear or branched. Also included herein are salts of these fatty acids. More preferably, the fatty acids are selected from the group consisting of lauric acid, palmitic acid, stearic acid, behenic acid and sebacic acid.

**In** one embodiment, the fatty alcohol derivatives and the fatty acid derivatives are preferably selected from the group consisting of alkyl ethers of fatty alcohols, alkoxylated fatty alcohols, alkyl ethers of alkoxylated fatty alcohols, esters of fatty alcohols, fatty acid esters of compounds having esterifiable hydroxy groups and hydroxy-substituted fatty acids. More preferably, the fatty alcohol derivatives and fatty acid derivatives are selected from the group consisting of methyl stearyl ether; the ceteth series of compounds such as ceteth-1 through ceteth-45, which are ethylene glycol ethers of cetyl alcohol, wherein the numeric designation indicates the number of ethylene glycol moieties present; the steareth series of compounds such as steareth-1 through 10, which are ethylene glycol ethers of steareth alcohol, wherein the numeric designation indicates the number of ethylene glycol moieties present; ceteareth-1 through ceteareth-10, which are the ethylene glycol ethers of ceteareth alcohol, i.e. a mixture of fatty alcohols containing predominantly cetyl and stearyl alcohol, wherein the numeric designation indicates the number of ethylene glycol moieties present; C1-C30 alkyl ethers of the ceteth, steareth, and ceteareth compounds; polyoxyethylene ethers of behenyl alcohol; ethyl stearate, cetyl stearate, cetyl palmitate, stearyl stearate, myristyl myristate, polyoxyethylene cetyl ether stearate, polyoxyethylene stearyl ether stearate, polyoxyethylene lauryl ether stearate, ethylene glycol monostearate, polyoxyethylene monostearate, polyoxyethylene distearate, propyleneglycol monostearate, propyleneglycol distearate, trimethylolpropane distearate, sorbitan stearate, polyglyceryl stearate, glyceryl monostearate, glyceryl distearate and glyceryl tristearate.

In one embodiment, the at least one component (B2) is present in an amount in the range of ≥ 1.0 to ≤ 10.0 wt.%, more preferably in the range of ≥ 2.0 to ≤ 9.0 wt.%, even more preferably in the range of ≥ 3.0 to ≤ 8.0 wt.%, most preferably in the range of ≥ 4.0 to ≤ 7.0 wt.%, in particular in the range of ≥ 5.0 to ≤ 6.0 wt.%, based on the total weight of the hair conditioning composition.

In one embodiment, the weight ratio of the at least one component (B2) to the at least one component (B1) is in the range of ≥ 1.0:1.0 to ≤ 5.0:1.0, more preferably in the range of ≥ 2.0:1.0 to ≤ 4.0:1.0.

In one embodiment, the gel matrix comprises an aqueous carrier (C) comprising water and C1-C6 alcohols. In one embodiment, the C1-C6 alcohols are preferably selected from the group consisting of ethanol, isopropanol, propylene glycol, hexylene glycol, glycerin and propane diol.

In a preferred embodiment, the conditioning composition preferably comprises at least one cosmetically acceptable adjuvant (D) selected from the group consisting of hydrolysed collagen, vitamin E, panthenol, panthenyl ethyl ether, hydrolysed keratins, proteins, peptides, plant extracts, nutrients, emollients, preservatives, pH-adjusting agents, electrolytes, coloring agents, perfumes, sequestering agents, ultraviolet and infrared screening and absorbing agents and antidandruff agents.

In a preferred embodiment, preservatives are selected from the group consisting of benzyl alcohol, methyl paraben, propyl paraben, imidazolidinyl urea, sodium benzoate, potassium sorbate, salicylic acid, methylchloroisothiazolinone and methylisothiazolinone.

In a preferred embodiment, anti-dandruff agents are selected from the group consisting of climbazole, octopirox and zinc pyrithione, salicylic acid, elemental sulfur, selenium dioxide, and the azole antimycotics. In a preferred embodiment, the anti-dandruff agent is present in the hair conditioning composition of the presently claimed invention in an amount in the range of ≥ 0.1 to ≤ 1.0 wt.%, more preferably in the range of ≥ 0.2 to ≤ 0.9 wt.%, even more preferably in the range of ≥ 0.3 to ≤ 0.8, 0.7, 0.6. or 0.5 wt.%, based on the total weight of the hair conditioning composition.

In a preferred embodiment, the pH adjusting agents are selected from the group consisting of citric acid, succinic acid, phosphoric acid, sodium hydroxide, and sodium carbonate; more preferably the pH adjusting agent is citric acid. In a preferred embodiment, the pH adjusting agent is present in an amount in the range of ≥ 0.1 to ≤ 0.5 wt.%, based on the total weight of the hair conditioning composition.

In a preferred embodiment, the term "electrolyte" refers to an ionic salt which is totally soluble in the hair conditioning composition of the presently claimed invention at the concentrations used. The electrolyte is preferably selected from the group consisting of alkali salts and ammonium salts, more preferably the electrolyte is an alkali salt such as NaCl or KCI. In a preferred embodiment, the electrolyte is present in an amount in the range of ≥ 0.1 to ≤ 0.3 wt.%, based on the total weight of the hair conditioning composition.

In a preferred embodiment, the hair conditioning composition includes a perfume obtained from a natural or a synthetic source. The perfume may be used along with a suitable solvent, diluents or carrier. Perfumes may be added in any conventionally known method, for example, admixing to a composition or blending with other ingredients used to form a composition, in amounts which are found to be useful to increase or impart the desired scent characteristics to hair conditioning compositions. Perfumes for the present application can be one or more selected from the following non-limiting group of compounds such as essential oils, absolutes, resinoids, resins, concretes, alcohols, aldehydes, ketones, ethers, acids, esters, acetals, ketals, nitriles, including saturated and unsaturated compounds and aliphatic, carbocyclic and heterocyclic compounds.

In yet another embodiment, the hair conditioning composition comprises sequestering agents. The term "sequestering agent" as used herein relates to a compound which is capable of bonding or complexing a metal ion between two or more atoms of the compound, thereby neutralizing or controlling harmful effects of such metal ions, wherein holding or bonding of a metal ion is through combination of one or more different types of bonds including coordination and/or ionic bonds. The suitable organic or inorganic sequestering agents are selected from the group consisting of polyols, gluconates, sorbitols, mannitols, carbonates, hydroxamates, catechols, alkanolamines, metal-ion sequestrants, hydroxy-carboxylic acids, aminocarboxylic acids, amino polycarboxylic acids, polyamines, polyphosphates, phosphonic acids, crown ethers, amino acids, polycarboxylic acids, cyclodextrin, phosphonates, polyacrylates or polymeric polycarboxylates and condensed phosphates.

In a preferred embodiment, the hair conditioning composition comprises a protein, more preferably a hydrolyzed cationic or non-cationic protein. Examples of these compounds include hydrolyzed collagens having trimethyl ammonium and trimethyl stearyl ammonium chloride groups, hydrolyzed animal proteins having trimethyl benzyl ammonium groups (benzyltrimonium hydrolyzed animal protein), hydrolyzed proteins having groups of quaternary ammonium on the polypeptide chain. Ceramide type of compounds include a ceramide, a glycoceramide, a pseudoceramide, or a neoceramide.

In a preferred embodiment, the hair conditioning composition comprises vitamins or their derivatives, such as vitamin B complex, including thiamine, nicotinic acid, biotin, pantothenic acid, choline, riboflavin, vitamin B6, vitamin B12, pyridoxine, inositol, carnitine, vitamins A, C, D, E, K and their derivatives, such as vitamin A palmitate, and pro-vitamins, e.g., panthenol (pro vitamin B5), panthenol triacetate and mixtures thereof.

In a preferred embodiment, suitable antioxidants may be added to facilitate the enhanced shelf-life of the hair conditioning composition of the presently claimed invention. The antioxidants that can be used include vitamins such as vitamin E, vitamin E acetate, vitamin C, vitamin A, and vitamin D, and derivatives thereof. Additional exemplary antioxidants include but are not limited to propyl, octyl and dodecyl esters of gallic acid, butylated hydroxyanisole (BHA), butylated hydroxytoluene (BHT), and nordihydroguaiaretic acid.

In a preferred embodiment, the hair conditioning composition comprises a plant extract selected from the group consisting of alnus, arnica, artemisia capillaris, Asia arum root, calendula, chamomile, conidium, comfrey, fennel, hawthorn, houttuynia, hypericum, jujube, kiwi, licorice, magnolia, olive, peppermint, philodendron and salvia.

In one embodiment, the hair conditioning composition is preferably free of cationic polysaccharides. Cationic polysaccharides are preferably selected from the group consisting of cationic celluloses and galactomannan gum; more preferably from the group consisting of cellulose ether derivatives comprising quaternary ammonium groups, cationic cellulose copolymers or cellulose derivatives grated with a water-soluble quaternary ammonium monomer and cationic galactomannan gums.

In one embodiment, the hair conditioning composition preferably comprises
at least one C3-C13 saturated or unsaturated dicarboxylic acid (E), which is unsubstituted or substituted with one hydroxyl group; and
at least one C10-C30 saturated or unsaturated monocarboxylic acid (F).

In one embodiment, the at least one component (E) is preferably selected from the group consisting of malonic acid, succinic acid, glutaric acid, adipic acid, malic acid, hydroxysuccinic acid, hydroxyglutaric acid, fumaric acid, maleic acid, itaconic acid, glutaconic acid and salts thereof. In one embodiment, the at least one component (E) is preferably present in an amount in the range of ≥ 0.1 to ≤ 2.0 wt.%, based on the total weight of the hair conditioning composition.

In a preferred embodiment, the at least one component (F) is selected from the group consisting of myristic acid, palmitoleic acid, stearic acid, behenic acid, oleic acid, linoleic acid, and isostearic acid; more preferably the at least one component (F) is oleic acid or palmitoleic acid. In one embodiment, the at least one component (F) is preferably present in an amount in the range of ≥ 0.05 to ≤ 1.0 wt.%, based on the total weight of the hair conditioning composition.

In one embodiment, the hair conditioning composition preferably has a pH in the range of ≥ 3.0 to ≤ 7.0.

In a preferred embodiment, the hair conditioning composition comprises
(A) ≥ 2.5 to ≤ 8 wt.% of a silicone system comprising
   (A1) at least one first non-volatile silicone selected from the group consisting of polydimethylsiloxanes, polydiethylsiloxanes and polymethylphenylsiloxanes having a viscosity at 25 °C in the range of ≥ 40,000 to ≤ 90,000 mm2/s, determined according to DIN 053019, which is free of amino groups;
   (A2) at least one second non-volatile silicone selected from the group consisting of polydimethylsiloxanes, polydiethylsiloxanes and polymethylphenylsiloxanes having a viscosity at 25 °C in the range of ≥ 10 to ≤ 1,000 mm2/s, determined according to DIN 053019, which is free of amino groups;
   (A3) at least one aminosilicone selected from the group consisting of Quaternium 80 and bisaminopropyl polydimethylsiloxane;
(B) a gel matrix comprising
   (B1) ≥ 1.0 to ≤ 4.0 wt.% of at least one cationic surfactant selected from the group consisting of cetrimonium halide, stearimonium halide, behentrimonium halide, dodecyltrimethylammonium halide, didodecyldimethylammonium halide, tetradecyltrimethylammonium halide, distearyldimethylammonium halide, dicetyldimethylammonium halide, behenamidopropyldimethylamine, behenamidopropyldiethylamine, behenamidoethyldiethyl-amine, behenamidoethyldimethylamine, arachidamidopropyldimethylamine, arachidamido-propyidiethylamine, arachidamidoethyidiethylamine and arachidamidoethyidimethylamine;
   (B2) ≥ 1.0 to ≤ 10.0 wt.% of at least one fatty compound selected from the group consisting of cetyl alcohol, stearyl alcohol and behenyl alcohol; and
(C) at least one aqueous carrier;
wherein each of the wt.% are based on the total weight of the hair conditioning composition.

In a preferred embodiment, the hair conditioning composition comprises
(A) ≥ 2.5 to ≤ 8 wt.% of a silicone system comprising
   (A1) at least one first non-volatile silicone selected from the group consisting of polydimethylsiloxanes, polydiethylsiloxanes and polymethylphenylsiloxanes having a viscosity at 25 °C in the range of ≥ 40,000 to ≤ 90,000 mm2/s, determined according to DIN 053019, which is free of amino groups;
   (A2) at least one second non-volatile silicone selected from the group consisting of polydimethylsiloxanes, polydiethylsiloxanes and polymethylphenylsiloxanes having a viscosity at 25 °C in the range of ≥ 10 to ≤ 1,000 mm2/s, determined according to DIN 053019, which is free of amino groups;
   (A3) Quaternium 80 and/or bisaminopropyl polydimethylsiloxanes;
(B) a gel matrix comprising
   (B1) ≥ 1.0 to ≤ 4.0 wt.% of at least one cationic surfactant selected from the group consisting of cetrimonium halide, stearimonium halide, behentrimonium halide, dodecyltrimethylammonium halide, didodecyldimethylammonium halide, tetradecyltrimethylammonium halide, distearyldimethylammonium halide, dicetyldimethylammonium halide, behenamidopropyldimethylamine, behenamidopropyldiethylamine, behenamidoethyldiethyl-amine, behenamidoethyldimethylamine, arachidamidopropyldimethylamine, arachidamido-propyidiethylamine, arachidamidoethyidiethylamine and arachidamidoethyidimethylamine;
   (B2) ≥ 1.0 to ≤ 10.0 wt.% of at least one fatty compound selected from the group consisting of cetyl alcohol, stearyl alcohol and behenyl alcohol; and
(C) at least one aqueous carrier;
wherein each of the wt.% are based on the total weight of the hair conditioning composition.

In another preferred embodiment, the hair conditioning composition comprises
(A) ≥ 2.0 to ≤ 10 wt.% of a silicone system comprising
   (A1) at least one first non-volatile silicone selected from the group consisting of polydimethylsiloxanes, polydiethylsiloxanes and polymethylphenylsiloxanes having a viscosity at 25 °C in the range of ≥ 40,000 to ≤ 90,000 mm2/s, determined according to DIN 053019, which is free of amino groups;
   (A2) at least one second non-volatile silicone selected from the group consisting of polydimethylsiloxanes, polydiethylsiloxanes and polymethylphenylsiloxanes having a viscosity at 25 °C in the range of ≥ 100 to ≤ 1,000 mm2/s, determined according to DIN 053019, which is free of amino groups;
   (A3) Quaternium 80 and/or bisaminopropyl polydimethylsiloxanes;
(B) a gel matrix comprising
   (B1) ≥ 0.1 to ≤ 20 wt.% of behentrimonium chloride;
   (B2) ≥ 0.1 to ≤ 20 wt.% of at least one fatty compound selected from the group consisting of cetyl alcohol, stearyl alcohol and behenyl alcohol having a melting point of ≥ 25 °C; and
(C) at least one aqueous carrier; wherein each of the wt.% is based on the total weight of the hair conditioning composition.

In another aspect, the presently claimed invention is directed to a method for reducing the hair volume comprising the steps of
a) treating the hair with a hair conditioning composition as defined herein, and
b) allowing the hair conditioning composition to contact the hair for a period in the range of ≥ 1 to ≤ 60 minutes, before the hair is dried.

In one embodiment, the method preferably results in hair frizz reduction.

In one embodiment, the method preferably results in hair shape retention.

In another aspect, the presently claimed invention is directed the use of the hair conditioning composition as defined herein for reducing hair volume.

### Advantages

The hair conditioning composition of the presently claimed invention is associated with one or more of the following advantages:
- The composition of the presently claimed invention allows for the provision of hair with improved manageability.
- The composition of the presently claimed invention allows for the provision of hair with long lasting style.
- The composition of the presently claimed invention allows for controlling the frizz of hair.
- The composition of the presently claimed invention imparts a smooth surface to the hair.
- The composition of the presently claimed invention reduces unwanted volume of hair.
- The composition of the presently claimed invention increases the shine of the hair.
- All of the above identified advantages are observed on both damaged and non-damaged hair.

### Embodiments

In the following, there is provided a list of embodiments to illustrate the present disclosure without intending to limit the disclosure to the specific embodiments listed below.

While the presently claimed invention has been described in terms of its specific embodiments, certain modifications and equivalents will be apparent to those skilled in the art and are intended to be included within the scope of the presently claimed invention.
1. A hair conditioning composition comprising
   (A) ≥ 2.0 to ≤ 10 wt.% of a silicone system comprising
      (A1) at least one first non-volatile silicone having a viscosity at 25 °C in the range of ≥ 40,000 to ≤ 90,000 mm2/s, determined according to DIN 053019, which is free of amino groups;
      (A2) at least one second non-volatile silicone having a viscosity at 25 °C in the range of ≥ 100 to ≤ 1,000 mm2/s, determined according to DIN 053019, which is free of amino groups;
      (A3) at least one aminosilicone selected from the group consisting of Quaternium 80 and bisaminopropyl polydimethylsiloxane;
   (B) a gel matrix comprising
      (B1) ≥ 0.1 to ≤ 20 wt.% of at least one cationic surfactant selected from the group consisting of quaternary ammonium compounds and amidoamines;
      (B2) ≥ 0.1 to ≤ 20 wt.% of at least one fatty compound having a melting point of ≥ 25 °C; and
   (C) at least one aqueous carrier;
   wherein each of the wt.% is based on the total weight of the hair conditioning composition.
2. The hair conditioning composition according to embodiment 1, wherein the silicone system (A) is present in an amount in the range of ≥ 2.5 to ≤ 8.0 wt.%, based on the total weight of the hair conditioning composition.
3. The hair conditioning composition according to embodiment 1 or 2, wherein the at least one first non-volatile silicone (A1) is selected from the group consisting of polydimethylsiloxanes, polydiethylsiloxanes and polymethylphenylsiloxanes.
4. The hair conditioning composition according to any one of embodiments 1 to 3, wherein the at least one component (A1) has a viscosity at 25 °C in the range of ≥ 45,000 to ≤ 85,000 mm2/s, determined according to DIN 053019.
5. The hair conditioning composition according to any one of embodiments 1 to 4, wherein the at least one component (A1) is present in an amount in the range of ≥ 1.0 to ≤ 5.0 wt.%, based on the total weight of the hair conditioning composition.
6. The hair conditioning composition according to any one of embodiments 1 to 5, wherein the at least one component (A2) is selected from the group consisting of polydimethylsiloxanes, polydiethylsiloxanes and polymethylphenylsiloxanes.
7. The hair conditioning composition according to any one of embodiments 1 to 6, wherein the at least one component (A2) has a viscosity at 25 °C in the range of ≥ 200 to ≤ 900 mm2/s, determined according to DIN 053019.
8. The hair conditioning composition according to any one of embodiments 1 to 7, wherein the at least one component (A2) is present in an amount in the range of ≥ 1.0 to ≤ 5.0 wt.%, based on the total weight of the hair conditioning composition.
9. The hair conditioning composition according to any one of embodiments 1 to 8, wherein the weight ratio of the at least one component (A1) to the at least one component (A2) is in the range of ≥ 0.5:1.0 to ≤ 2.0:1.0.
10. The hair conditioning composition according to any one of embodiments 1 to 9, wherein the at least one component (A3) is selected from the group consisting of Quaternium 80 and bisaminopropyl polydimethylsiloxane.
11. The hair conditioning composition according any one of embodiments 1 to 10, wherein the at least one component (A3) has a weight average molecular weight M_{w} in the range from ≥ 2,000 to ≤ 50,000 g/mol.
12. The hair conditioning composition according to any one of embodiments 1 to 11, wherein the at least one component (A3) is present in an amount in the range of ≥ 0.5 to ≤ 6.0 wt.%, based on the total weight of the hair conditioning composition.
13. The hair conditioning composition according to any one of embodiments 1 to 12, wherein the weight ratio of the total amount of the at least one component (A1) and the at least one component (A2) to the at least one component (A3) is in the range of ≥ 4.0:1.0 to ≤ 1.0:1.0.
14. The hair conditioning composition according to any one of embodiments 1 to 12, wherein the quaternary ammonium compound has a general formula (I) 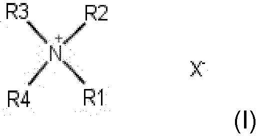 wherein
   - R1: is selected from the group consisting of linear or branched, substituted or unsubstituted C1-C30 alkyl, linear or branched, substituted or unsubstituted C2-C30 alkenyl, substituted or unsubstituted, linear or branched C1-C30 heteroalkyl and substituted or unsubstituted, linear or branched C2-C30 heteroalkenyl;
   - R2: is selected from the group consisting of linear or branched, substituted or unsubstituted C6-C30 alkyl, linear or branched, substituted or unsubstituted C6-C30 alkenyl, substituted or unsubstituted, linear or branched C6-C30 heteroalkyl and substituted or unsubstituted, linear or branched C6-C30 heteroalkenyl;
   - R3 and R4: are, identical or different, are selected from the group consisting of linear or branched, substituted or unsubstituted C1-C30 alkyl, linear or branched, substituted or unsubstituted C2-C30 alkenyl, substituted or unsubstituted C6-C30 aryl and substituted or unsubstituted C1-C10 alkylene C6-C30 aryl; and
   - X⁻: is selected from the group consisting of halide, alkyl sulfates, phosphates, alkyl sulfonates, alkylaryl sulfonates and anions derived from organic acids and amino acids.
   14. The hair conditioning composition according to embodiment 13, wherein
   - R1: is linear or branched, unsubstituted C1-C30 alkyl;
   - R2: is linear or branched, unsubstituted C6-C30 alkyl;
   - R3 and R4: are, identical or different, linear or branched, unsubstituted C1-C4 alkyl; and
   - X⁻: is halide.
15. The hair conditioning composition according to embodiment 13 or 14, wherein the quaternary ammonium compounds are selected from the group consisting of cetrimonium halide, stearimonium halide, behentrimonium halide, dodecyltrimethylammonium halide, didodecyldimethylammonium halide, tetradecyltrimethylammonium halide, distearyldimethylammonium halide and dicetyldimethylammonium halide.
16. The hair conditioning composition according to any one of embodiments 1 to 15, wherein the amidoamine has a general formula (II),

   R5-C(=O)-NH-(CH₂)n-NR6R7 (II),

   wherein
   - R5: is selected from the group consisting of linear or branched, substituted or unsubstituted C1-C30 alkyl, linear or branched, substituted or unsubstituted C2-C30 alkenyl,
   - R3 and R4: are, identical or different, are selected from the group consisting of linear or branched, substituted or unsubstituted C6-C30 alkyl, linear or branched, substituted or unsubstituted C6-C30 alkenyl, substituted or unsubstituted, linear or branched C6-C30 heteroalkyl, substituted or unsubstituted, linear or branched C6-C30 heteroalkenyl, substituted or unsubstituted C6-C30 aryl and substituted or unsubstituted C1-C10 alkylene C6-C30 aryl; and
   - n: is 1, 2, 3 or 4.
17. The hair conditioning composition according to embodiment 16, wherein the amidoamine is selected from the group consisting of
   behenamidopropyldimethylamine, behenamidopropyldiethylamine, behenamidoethyldiethylamine, behenamidoethyldimethylamine, arachidamidopropyldimethylamine, arachidamidopropyldiethylamine, arachidamidoethyidiethylamine and arachidamidoethyidimethylamine.
18. The hair conditioning composition according to any one of embodiments 1 to 17, wherein the at least one component (B1) is present in an amount in the range of ≥ 1.0 to ≤ 4.0 wt.%, based on the total weight of the hair conditioning composition.
19. The hair conditioning composition according to any one of embodiments 1 to 18, wherein the at least one component (B2) is selected from the group consisting of fatty alcohols, fatty acids, fatty alcohol derivatives and fatty acid derivatives.
20. The hair conditioning composition according to embodiment 19, wherein the fatty alcohols are selected from the group consisting of cetyl alcohol, stearyl alcohol and behenyl alcohol.
21. The hair conditioning composition according to any one of embodiments 1 to 20, wherein the at least one component (B2) is present in an amount in the range of ≥ 1.0 to ≤ 10.0 wt.%, based on the total weight of the hair conditioning composition.
22. The hair conditioning composition according to any one of embodiments 1 to 21, wherein the weight ratio of the at least one component (B2) to the at least one component (B1) is in the range of ≥ 1.0:1.0 to ≤ 5.0:1.0.
23. The hair conditioning composition according to any one of embodiments 1 to 22, wherein the aqueous carrier (C) comprises water and C₁-C₆ alcohols.
24. The hair conditioning composition according to any one of embodiments 1 to 23, wherein the hair conditioning composition comprises at least one cosmetically acceptable adjuvant (D) selected from the group consisting of hydrolysed collagen, vitamin E, panthenol, panthenyl ethyl ether, hydrolysed keratins, proteins, peptides, plant extracts, nutrients, emollients, preservatives, pH-adjusting agents, electrolytes, coloring agents, perfumes, sequestering agents, ultraviolet and infrared screening and absorbing agents and antidandruff agents.
25. The hair conditioning composition according to any one of embodiments 1 to 24, wherein the hair conditioning composition is free of cationic polysaccharides.
26. The hair conditioning composition according to any one of embodiments 1 to 25, wherein the hair conditioning composition comprises
   at least one C3-C13 saturated or unsaturated dicarboxylic acid (E), which is unsubstituted or substituted with one hydroxyl group; and
   at least one C10-C30 saturated or unsaturated monocarboxylic acid (F).
27. The hair conditioning composition according to embodiment 26, wherein the at least one component (E) is selected from the group consisting of malonic acid, succinic acid, glutaric acid, adipic acid, malic acid, hydroxysuccinic acid, hydroxyglutaric acid, fumaric acid, maleic acid, itaconic acid, glutaconic acid and salts thereof.
28. The hair conditioning composition according to embodiment 26 or 27, wherein the at least one component (E) is present in an amount in the range of ≥ 0.1 to ≤ 2.0 wt.%, based on the total weight of the hair conditioning composition.
29. The hair conditioning composition according to embodiment 26, wherein at least one component (F) is oleic acid or palmitoleic acid.
30. The hair conditioning composition according to any one of embodiments 26 to 29, wherein the at least one component (F) is present in an amount in the range of ≥ 0.05 to ≤ 1.0 wt.%, based on the total weight of the hair conditioning composition.
31. The hair conditioning composition according to any one of embodiments 1 to 30, wherein the hair conditioning composition has a pH in the range of ≥ 3.0 to ≤ 7.0.
32. The hair conditioning composition according to any one of embodiments 1 to 31 comprising
   (A) ≥ 2.5 to ≤ 8 wt.% of a silicone system comprising
      (A1) at least one first non-volatile silicone selected from the group consisting of polydimethylsiloxanes, polydiethylsiloxanes and polymethylphenylsiloxanes having a viscosity at 25 °C in the range of ≥ 40,000 to ≤ 90,000 mm2/s, determined according to **DIN** 053019, which is free of amino groups;
      (A2) at least one second non-volatile silicone selected from the group consisting of polydimethylsiloxanes, polydiethylsiloxanes and polymethylphenylsiloxanes having a viscosity at 25 °C in the range of ≥ 10 to ≤ 1,000 mm2/s, determined according to **DIN** 053019, which is free of amino groups;
      (A3) at least one aminosilicone selected from the group consisting of Quaternium 80 and bisaminopropyl polydimethylsiloxane;
   (B) a gel matrix comprising
      (B1) ≥ 1.0 to ≤ 4.0 wt.% of at least one cationic surfactant selected from the group consisting of cetrimonium halide, stearimonium halide, behentrimonium halide, dodecyltrimethylammonium halide, didodecyldimethylammonium halide, tetradecyltrimethylammonium halide, distearyldimethylammonium halide, dicetyldimethylammonium halide, behenamidopropyldimethylamine, behenamidopropyldiethylamine, behenamidoethyldiethylamine, behenamidoethyldimethylamine, arachidamidopropyldimethylamine, arachidamido-propyidiethylamine, arachidamidoethyidiethylamine and arachidamidoethyidimethylamine;
      (B2) ≥ 1.0 to ≤ 10.0 wt.% of at least one fatty compound selected from the group consisting of cetyl alcohol, stearyl alcohol and behenyl alcohol; and
   (C) at least one aqueous carrier;
   wherein each of the wt.% are based on the total weight of the hair conditioning composition.
32. A method for reducing hair volume comprising the steps of
   a) treating the hair with a hair conditioning composition according to any one of embodiments 1 to 31, and
   b) allowing the hair conditioning composition to contact the hair for a period in the range of ≥ 1 to ≤ 60 minutes, before the hair is dried.
33. The method according to embodiment 32, wherein the method results in hair frizz reduction.
34. The method according to embodiment 32 or 333, wherein the method results in hair shape retention.
35. Use of a hair conditioning composition according to any one of embodiments 1 to 31 for reducing hair volume.

### Examples

The presently claimed invention is illustrated in detail by non-restrictive working examples which follow. More particularly, the test methods specified hereinafter are part of the general disclosure of the application and are not restricted to the specific working examples.

### Materials

- Cetyl alcohol, commercially available from i.e from GODREJ INDUSTRIES LIMITED (Ginol^{®} 16)
- Stearyl alcohol, commercially available from i.e from GODREJ INDUSTRIES LIMITED (Ginol^{®} 18)
- Behentrimonium chloride, commercially available from i.e. from Clariant (Genamin^{®} KDMP)
- Phenoxyethanol, commercially available from i.e. from DuPont (NEOLONE^{™} PH 100)
- Disodium EDTA, commercially available from Nouryon (Dissolvine^{®} NA2-S)
- Methylparaben, commercially available from Clariant (Nipagin^{®} M)
- Propylparaben, commercially available from Clariant (Nipasol^{®} M)
- Quaternium 80, commercially available from Evonik (ABIL^{®} Quat 3272)
- Dimethicone having a viscosity at 25 °C of 60,000 mm2/s, determined according to DIN 053019, commercially available from Dow Corning (XIAMETER^{™} PMX-200 Silicone Fluid 60,000 cSt)
- Dimethicone having a viscosity at 25 °C of 500 mm2/s, determined according to DIN 053019, commercially available from XIAMETER^{™} PMX-200 Silicone Fluid 500 cSt
- Dimethicone having a viscosity at 25 °C of 10,000 mm2/s, determined according to DIN 053019, commercially available from XIAMETER^{™} PMX-200 Silicone Fluid 10,000 cSt
- Dimethicone having a viscosity at 25 °C of 100,000 mm2/s, determined according to DIN 053019, commercially available from XIAMETER^{™} PMX-200 Silicone Fluid 100,000 cSt

### Preparation of hair conditioning compositions

The exemplified compositions can be prepared by conventional formulation and mixing techniques. It will be appreciated that other modifications of the present invention within the skill of those in the hair care formulation art can be undertaken without departing from the spirit and scope of this invention. All parts, percentages, and ratios herein are by weight unless otherwise specified. Some components may come from suppliers as dilute solutions.

**Table 1.**

| | **Composition a)*** | **Composition b)*** | **Composition c)** |
|---|---|---|---|
| Cetyl alcohol | 1.59 | 1.59 | 1.59 |
| Stearyl alcohol | 4.05 | 4.05 | 4.05 |
| Behentrimonium chloride | 2.28 | 2.28 | 2.28 |
| Phenoxyethanol | 0.5 | 0.5 | 0.5 |
| Disodium EDTA | 0.1 | 0.1 | 0.1 |
| Methylparaben | 0.2 | 0.2 | 0.2 |
| Propylparaben | 0.15 | 0.15 | 0.15 |
| Quaternium 80 | 1.5 | 1.5 | 1.5 |
| Dimethicone 60000 mm2/s | - | 4 | 2 |
| Dimethicone 500 mm2/s | 4 | - | 2 |
| Perfume | 0.5 | 0.5 | 0.5 |
| Water Purified | ad 100 | ad 100 | ad 100 |
| Sum | 100 | 100 | 100 |

| | | | |
|---|---|---|---|
| *not within the scope of the presently claimed invention | | | |

**Table 2**

| | **Composition d*** | **Composition e** | **Composition f*** |
|---|---|---|---|
| Raw Materials: | | | |
| Water Purified | ad 100 | ad 100 | ad 100 |
| Behentrimonium Chloride (in Isopropanol) | 2.28 | 2.28 | 2.28 |
| PHENOXYETHANOL | 0.9 | 0.9 | 0.9 |
| Cetyl Alcohol 95% | 1.59 | 1.59 | 1.59 |
| Stearyl Alcohol 97% | 4.05 | 4.05 | 4.05 |
| Quaternium-80 | 1.5 | 1.5 | 1.5 |
| Fragrance | 0.5 | 0.5 | 0.5 |
| 500 mm2/s-1 Dimethicone | 2.0 | 2.0 | 2.0 |
| 10,000 mm2/s-1 Dimethicone | 2.0 | - | - |
| 60,000 mm2/s-1 Dimethicone | - | 2.0 | - |
| 100,000 mm2/s-1 Dimethicone | - | - | 2.0 |

### Test methods

### Hair Volume and Hair Body Measurement

The definitions of hair volume and body were essentially based on visual perception ('volume') and tactile sensation ('body'). The visually perceptible volume was measured by image analysis, e.g. by taking the shadow which the hair tress to be tested casted, when suitably illuminated. Another measure for the hair volume was the projection area based on images of a tress photographed from four sides (cf. A. Böther, B. Primmel, W. Seidel, Haarkosmetisch bedingte Volumenänderungen bestimmen, Parfümerie und Kosmetik 79 (10), 26 (1998)).

The methods applied were suited to determine both the visual and the tactile volumes.

### Hair Body Test (Tactile Volume)

A hair tress was pulled through a metal ring having a diameter of 20mm (examples 5 to 8) or 12 mm (examples 9 to 12) and the force which had to be exerted was measured. The bigger the volume of the tress, the more force was required. Higher resistance of the tress against pulling through indicates higher resistance against deformation (strengthening of the hairstyle, volume) (cf. C. R. Robbins, Chemical and Physical Behavior of Human Hair, 5th Edition, p. 686 ff., Springer-Verlag, Berlin Heidelberg 2012. ISBN 978-3-642-25610-3).

### Visible Volume Test

The hair tress was illuminated from one side and a CCD camera positioned at the opposite side took images while the tress was rotated automatically. With the images taken from all sides of the tress a 3-dimensional shape was calculated. It represented the total Visible Volume.

### Hair tresses preparation for the hair volume and body measurements

Hair tresses (wavy Chinese hair volume tresses), weight 2.85g, 17cm long, were bleached to simulate hair damage and then soaked in municipal water for 10 min (35°C ± 2°C). Afterwards, the tresses were adjusted to 50% rel. humidity by weight, then shampooed with a neutral shampoo (0.25 ml NC Shampoo per g hair was applied to the tresses and massaged in for 1 min.) and then rinsed off 1 minute with municipal water (6L/min, 35°C). Afterwards, 0.25 ml of the conditioner examples per g hair were applied to the tresses and massaged in for 1 min. Application time was 5 min at room temperature. The tresses were rinsed off for 1 min with municipal water (6 L/min; 35°C) and were combed 3 times with the raw side and 3 times with the fine side of a metal comb. The tresses were formed into a round shape and fixed into metal sleeves and stored overnight in a climatic chamber (20°C; 65% r.h.) for the actual volume/body measurements.

### Results

The visible volume and body of hair tresses were measured (n=3) after shampooing with neutral shampoo and treating with the compositions a) to d).

**Table 3. Visible volume**

| Example | | **Visible Volume / cm³** |
|---|---|---|
| 1* | Shampoo only | 504 ± 34 |
| 2* | Shampoo and composition a*) | 470 ± 22 |
| 3* | Shampoo and composition b*) | 307 ± 33 |
| 4 | Shampoo and composition c) | 184 ± 33 |

| | | |
|---|---|---|
| *not within the scope of the presently claimed invention | | |

**Table 4. Body**

| Example | | **Hair Body/Nmm** |
|---|---|---|
| 5* | Shampoo only | 1.0 ± 0.09 |
| 6* | Shampoo and composition a*) | 1.03 ± 0.13 |
| 7* | Shampoo and composition b*) | 0.68 ± 0.07 |
| 8 | Shampoo and composition c) | 0.22 ± 0.11 |
| 9* | Shampoo only | 1.82 ± 0.09 |
| 10* | Shampoo and composition d*) | 0.58 ± 0.10 |
| 11 | Shampoo and composition e) | 0.33 ± 0.14 |
| 12* | Shampoo and composition f*) | 0.42 ± 0.09 |

| | | |
|---|---|---|
| *not within the scope of the presently claimed invention | | |

### Discussion of results

The results indicate clearly that compositions containing two non-volatile silicones with low and high viscosity in combination with an aminosilicone in a gel matrix (examples 4 and 8) resulted in better hair manageability/volume reduction versus the combination of a single dimethicone with an aminosilicone (Examples 2, 3, 5, 7) in the same gel matrix. Thus, the combination of non-volatile silicones with low and high viscosity and an aminosilicone in a gel matrix showed a beneficial effect on hair manageability and reduction of hair volume.

The results indicate clearly that compositions containing two non-volatile silicones with low and high viscosity in combination with an aminosilicone in a gel matrix does result in best hair manageability/volume reduction performance when the viscosity of the high viscosity silicone is about 60,000 mm2/s-1 (claim 1.A1 in EP22196994.2). Surprisingly, a much lower or much higher viscosity (10,000 respectively 100,000 mm2/s-, examples 10 and 12) does result in a worse hair manageability/volume reduction performance versus the medium viscosity silicone (60,000 mm2/s-1).

Therefore, the silicone combination described in US7887787B2, which contains an high viscosity silicone in the viscosity range of 300,000 - 700,000 mm2/s-1, does deliver a worse hair manageability/volume reduction performance versus the silicone combination described in EP22196994.2.

## Claims

1. A hair conditioning composition comprising
(A) ≥ 2.0 to ≤ 10 wt.% of a silicone system comprising
(A1) at least one first non-volatile silicone having a viscosity at 25 °C in the range of ≥ 40,000 to ≤ 90,000 mm2/s, determined according to DIN 053019, which is free of amino groups;
(A2) at least one second non-volatile silicone having a viscosity at 25 °C in the range of ≥ 100 to ≤ 1,000 mm2/s, determined according to DIN 053019, which is free of amino groups;
(A3) at least one aminosilicone selected from the group consisting of Quaternium 80 and bisaminopropyl polydimethylsiloxane;
(B) a gel matrix comprising
(B1) ≥ 0.1 to ≤ 20 wt.% of at least one cationic surfactant selected from the group consisting of quaternary ammonium compounds and amidoamines;
(B2) ≥ 0.1 to ≤ 20 wt.% of at least one fatty compound having a melting point of ≥ 25 °C; and
(C) at least one aqueous carrier;
wherein each of the wt.% is based on the total weight of the hair conditioning composition.

2. The hair conditioning composition according to claim 1, wherein the silicone system (A) is present in an amount in the range of ≥ 2.5 to ≤ 8.0 wt.%, based on the total weight of the hair conditioning composition.

3. The hair conditioning composition according to claim 1 or 2, wherein the at least one first non-volatile silicone (A1) is selected from the group consisting of polydimethylsiloxanes, polydiethylsiloxanes and polymethylphenylsiloxanes.

4. The hair conditioning composition according to any one of claims 1 to 3, wherein the at least one component (A1) has a viscosity at 25 °C in the range of ≥ 45,000 to ≤ 85,000 mm2/s, determined according to DIN 053019.

5. The hair conditioning composition according to any one of claims 1 to 4, wherein the at least one component (A1) is present in an amount in the range of ≥ 0.8 to ≤ 5.0 wt.%, based on the total weight of the hair conditioning composition.

6. The hair conditioning composition according to any one of claims 1 to 5, wherein the at least one component (A2) is selected from the group consisting of polydimethylsiloxanes, polydiethylsiloxanes and polymethylphenylsiloxanes.

7. The hair conditioning composition according to any one of claims 1 to 6, wherein the at least one component (A2) has a viscosity at 25 °C in the range of ≥ 200 to ≤ 900 mm2/s, determined according to DIN 053019.

8. The hair conditioning composition according to any one of claims 1 to 7, wherein the at least one component (A2) is present in an amount in the range of ≥ 0.8 to ≤ 5.0 wt.%, based on the total weight of the hair conditioning composition.

9. The hair conditioning composition according to any one of claims 1 to 8, wherein the weight ratio of the at least one component (A1) to the at least one component (A2) is in the range of ≥ 0.5:1.0 to ≤ 2.0:1.0.

10. The hair conditioning composition according any one of claims 1 to 9, wherein the at least one component (A3) has a weight average molecular weight M_{w} in the range from ≥ 2,000 to ≤ 50,000 g/mol.

11. The hair conditioning composition according to any one of claims 1 to 10, wherein the at least one component (A3) is present in an amount in the range of ≥ 0.5 to ≤ 4.0 wt.%, based on the total weight of the hair conditioning composition.

12. The hair conditioning composition according to any one of claims 1 to 11, wherein the weight ratio of the total amount of the at least one component (A1) and the at least one component (A2) to the at least one component (A3) is in the range of ≥ 4.0:1.0 to ≤ 1.0:1.0.

13. The hair conditioning composition according to any one of claims 1 to 12, wherein the at least one component (B1) is selected from the group consisting of cetrimonium halide, stearimonium halide, behentrimonium halide, dodecyltrimethylammonium halide, didodecyldimethylammonium halide, tetradecyltrimethylammonium halide, distearyldimethylammonium halide, dicetyldimethylammonium halide, behenamidopropyldimethylamine, behenamidopropyldiethylamine, behenamidoethyldiethylamine, behenamidoethyldimethylamine, arachidamidopropyldimethylamine, arachidamidopropyldiethylamine, arachidamidoethyidiethylamine and arachidamidoethyidimethylamine.

14. The hair conditioning composition according to any one of claims 1 to 13, wherein the at least one component (B2) is selected from the group consisting of fatty alcohols, fatty acids, fatty alcohol derivatives and fatty acid derivatives.

15. The hair conditioning composition according to any one of claims 1 to 14, wherein the weight ratio of the at least one component (B2) to the at least one component (B1) is in the range of ≥ 1.0:1.0 to ≤ 5.0:1.0.

16. The hair conditioning composition according to any one of claims 1 to 15, wherein the hair conditioning composition is free of cationic polysaccharides.

17. A method for reducing hair volume comprising the steps of
a) treating the hair with a hair conditioning composition according to any one of claims 1 to 16, and
b) allowing the hair conditioning composition to contact the hair for a period in the range of ≥ 1 to ≤ 60 minutes, before the hair is dried.

18. Use of a hair conditioning composition according to any one of claims 1 to 16 for reducing hair volume.

## Patentansprüche

1. Haarkonditionierungszusammensetzung, umfassend
(A) ≥ 2,0 bis ≤ 10 Gew.-% eines Siliconsystems, umfassend
(A1) mindestens ein erstes nichtflüchtiges Silicon mit einer Viskosität bei 25 °C im Bereich von ≥ 40 000 bis ≤ 90 000 mm2/s, bestimmt nach DIN 053019, das frei von Aminogruppen ist;
(A2) mindestens ein zweites nichtflüchtiges Silicon mit einer Viskosität bei 25 °C im Bereich von ≥ 100 bis ≤ 1 000 mm2/s, bestimmt nach DIN 053019, das frei von Aminogruppen ist;
(A3) mindestens ein Aminosilicon, ausgewählt aus der Gruppe bestehend aus Quaternium 80 und Bisaminopropylpolydimethylsiloxan;
(B) eine Gelmatrix, umfassend
(B1) ≥ 0,1 bis ≤ 20 Gew.-% mindestens eines kationischen Tensids, ausgewählt aus der Gruppe bestehend aus quartären Ammoniumverbindungen und Amidoaminen;
(B2) ≥ 0,1 bis ≤ 20 Gew.-% mindestens einer Fettverbindung mit einem Schmelzpunkt von ≥ 25 °C; und
(C) mindestens einen wässrigen Träger;
wobei sich jedes der Gew.-% auf das Gesamtgewicht der Haarkonditionierungszusammensetzung bezieht.

2. Haarkonditionierungszusammensetzung nach Anspruch 1, wobei das Siliconsystem (A) in einer Menge im Bereich von ≥ 2,5 bis ≤ 8,0 Gew.-% vorliegt, bezogen auf das Gesamtgewicht der Haarkonditionierungszusammensetzung.

3. Haarkonditionierungszusammensetzung nach Anspruch 1 oder 2, wobei das mindestens eine erste nichtflüchtige Silicon (A1) ausgewählt ist aus der Gruppe bestehend aus Polydimethylsiloxanen, Polydiethylsiloxanen und Polymethylphenylsiloxanen.

4. Haarkonditionierungszusammensetzung nach einem der Ansprüche 1 bis 3, wobei die mindestens eine Komponente (A1) eine Viskosität bei 25 °C im Bereich von ≥ 45 000 bis ≤ 85 000 mm2/s aufweist, bestimmt nach DIN 053019.

5. Haarkonditionierungszusammensetzung nach einem der Ansprüche 1 bis 4, wobei die mindestens eine Komponente (A1) in einer Menge im Bereich von ≥ 0,8 bis ≤ 5,0 Gew.-% vorliegt, bezogen auf das Gesamtgewicht der Haarkonditionierungszusammensetzung.

6. Haarkonditionierungszusammensetzung nach einem der Ansprüche 1 bis 5, wobei die mindestens eine Komponente (A2) ausgewählt ist aus der Gruppe bestehend aus Polydimethylsiloxanen, Polydiethylsiloxanen und Polymethylphenylsiloxanen.

7. Haarkonditionierungszusammensetzung nach einem der Ansprüche 1 bis 6, wobei die mindestens eine Komponente (A2) eine Viskosität bei 25 °C im Bereich von ≥ 200 bis ≤ 900 mm2/s aufweist, bestimmt nach DIN 053019.

8. Haarkonditionierungszusammensetzung nach einem der Ansprüche 1 bis 7, wobei die mindestens eine Komponente (A2) in einer Menge im Bereich von ≥ 0,8 bis ≤ 5,0 Gew.-% vorliegt, bezogen auf das Gesamtgewicht der Haarkonditionierungszusammensetzung.

9. Haarkonditionierungszusammensetzung nach einem der Ansprüche 1 bis 8, wobei das Gewichtsverhältnis der mindestens einen Komponente (A1) zu der mindestens einen Komponente (A2) im Bereich von ≥ 0,5:1,0 bis ≤ 2,0:1,0 liegt.

10. Haarkonditionierungszusammensetzung nach einem der Ansprüche 1 bis 9, wobei die mindestens eine Komponente (A3) ein gewichtsmittleres Molekulargewicht Mw im Bereich von ≥ 2 000 bis ≤ 50 000 g/mol aufweist.

11. Haarkonditionierungszusammensetzung nach einem der Ansprüche 1 bis 10, wobei die mindestens eine Komponente (A3) in einer Menge im Bereich von ≥ 0,5 bis ≤ 4,0 Gew.-% vorliegt, bezogen auf das Gesamtgewicht der Haarkonditionierungszusammensetzung.

12. Haarkonditionierungszusammensetzung nach einem der Ansprüche 1 bis 11, wobei das Gewichtsverhältnis der Gesamtmenge der mindestens einen Komponente (A1) und der mindestens einen Komponente (A2) zu der mindestens einen Komponente (A3) im Bereich von ≥ 4,0:1,0 bis ≤ 1,0:1,0 liegt.

13. Haarkonditionierungszusammensetzung nach einem der Ansprüche 1 bis 12, wobei die mindestens eine Komponente (B1) ausgewählt ist aus der Gruppe bestehend aus Cetrimoniumhalogenid, Stearimoniumhalogenid, Behentrimoniumhalogenid, Dodecyltrimethylammoniumhalogenid, Didodecyldimethylammoniumhalogenid, Tetradecyltrimethylammoniumhalogenid, Distearyldimethylammoniumhalogenid, Dicetyldimethylammoniumhalogenid, Behenamidopropyldimethylamin, Behenamidopropyldiethylamin, Behenamidoethyldiethylamin, Behenamidoethyldimethylamin, Arachidamidopropyldimethylamin, Arachidamidopropyldiethylamin, Arachidamidoethyidiethylamin und Arachidamidoethyidimethylamin.

14. Haarkonditionierungszusammensetzung nach einem der Ansprüche 1 bis 13, wobei die mindestens eine Komponente (B2) ausgewählt ist aus der Gruppe bestehend aus Fettalkoholen, Fettsäuren, Fettalkoholderivaten und Fettsäurederivaten.

15. Haarkonditionierungszusammensetzung nach einem der Ansprüche 1 bis 14, wobei das Gewichtsverhältnis der mindestens einen Komponente (B2) zu der mindestens einen Komponente (B1) im Bereich von ≥ 1,0:1,0 bis ≤ 5,0:1,0 liegt.

16. Haarkonditionierungszusammensetzung nach einem der Ansprüche 1 bis 15, wobei die Haarkonditionierungszusammensetzung frei von kationischen Polysacchariden ist.

17. Verfahren zur Reduzierung des Haarvolumens, umfassend die Schritte
a) Behandeln des Haares mit einer Haarkonditionierungszusammensetzung nach einem der Ansprüche 1 bis 16, und
b) Belassen der Haarkonditionierungszusammensetzung in Kontakt mit dem Haar für einen Zeitraum im Bereich von ≥ 1 bis ≤ 60 Minuten, bevor das Haar getrocknet wird.

18. Verwendung einer Haarkonditionierungszusammensetzung nach einem der Ansprüche 1 bis 16 zur Reduzierung des Haarvolumens.

## Revendications

1. Composition de conditionnement capillaire, comprenant
(A) ≥ 2,0 à ≤ 10 % en poids d'un système de silicone comprenant
(A1) au moins un premier silicone non volatil ayant une viscosité à 25 °C dans la plage de ≥ 40 000 à ≤ 90 000 mm2/s, déterminée selon DIN 053019, qui est exempt de groupes amino ;
(A2) au moins un deuxième silicone non volatil ayant une viscosité à 25 °C dans la plage de ≥ 100 à ≤ 1 000 mm2/s, déterminée selon DIN 053019, qui est exempt de groupes amino ;
(A3) au moins un aminosilicone choisi dans le groupe constitué de Quaternium 80 et de bisaminopropylpolydiméthylsiloxane ;
(B) une matrice de gel comprenant
(B1) ≥ 0,1 à ≤ 20 % en poids d'au moins un tensioactif cationique choisi dans le groupe constitué des composés d'ammonium quaternaire et des amidoamines ;
(B2) ≥ 0,1 à ≤ 20 % en poids d'au moins un composé gras ayant un point de fusion de ≥ 25 °C ; et
(C) au moins un support aqueux ;
dans laquelle chacun des % en poids est rapporté au poids total de la composition de conditionnement capillaire.

2. Composition de conditionnement capillaire selon la revendication 1, dans laquelle le système de silicone (A) est présent en une quantité dans la plage de ≥ 2,5 à ≤ 8,0 % en poids, par rapport au poids total de la composition de conditionnement capillaire.

3. Composition de conditionnement capillaire selon la revendication 1 ou 2, dans laquelle l'au moins un premier silicone non volatil (A1) est choisi dans le groupe constitué des polydiméthylsiloxanes, des polydiéthylsiloxanes et des polyméthylphénylsiloxanes.

4. Composition de conditionnement capillaire selon l'une quelconque des revendications 1 à 3, dans laquelle l'au moins un composant (A1) a une viscosité à 25 °C dans la plage de ≥ 45 000 à ≤ 85 000 mm2/s, déterminée selon DIN 053019.

5. Composition de conditionnement capillaire selon l'une quelconque des revendications 1 à 4, dans laquelle l'au moins un composant (A1) est présent en une quantité dans la plage de ≥ 0,8 à ≤ 5,0 % en poids, par rapport au poids total de la composition de conditionnement capillaire.

6. Composition de conditionnement capillaire selon l'une quelconque des revendications 1 à 5, dans laquelle l'au moins un composant (A2) est choisi dans le groupe constitué des polydiméthylsiloxanes, des polydiéthylsiloxanes et des polyméthylphénylsiloxanes.

7. Composition de conditionnement capillaire selon l'une quelconque des revendications 1 à 6, dans laquelle l'au moins un composant (A2) a une viscosité à 25 °C dans la plage de ≥ 200 à ≤ 900 mm2/s, déterminée selon DIN 053019.

8. Composition de conditionnement capillaire selon l'une quelconque des revendications 1 à 7, dans laquelle l'au moins un composant (A2) est présent en une quantité dans la plage de ≥ 0,8 à ≤ 5,0 % en poids, par rapport au poids total de la composition de conditionnement capillaire.

9. Composition de conditionnement capillaire selon l'une quelconque des revendications 1 à 8, dans laquelle le rapport pondéral de l'au moins un composant (A1) à l'au moins un composant (A2) est dans la plage de ≥ 0,5:1,0 à ≤ 2,0:1,0.

10. Composition de conditionnement capillaire selon l'une quelconque des revendications 1 à 9, dans laquelle l'au moins un composant (A3) a une masse moléculaire moyenne en poids Mw dans la plage de ≥ 2 000 à ≤ 50 000 g/mol.

11. Composition de conditionnement capillaire selon l'une quelconque des revendications 1 à 10, dans laquelle l'au moins un composant (A3) est présent en une quantité dans la plage de ≥ 0,5 à ≤ 4,0 % en poids, par rapport au poids total de la composition de conditionnement capillaire.

12. Composition de conditionnement capillaire selon l'une quelconque des revendications 1 à 11, dans laquelle le rapport pondéral de la quantité totale de l'au moins un composant (A1) et de l'au moins un composant (A2) à l'au moins un composant (A3) est dans la plage de ≥ 4,0:1,0 à ≤ 1,0:1,0.

13. Composition de conditionnement capillaire selon l'une quelconque des revendications 1 à 12, dans laquelle l'au moins un composant (B1) est choisi dans le groupe constitué de l'halogénure de cetrimonium, de l'halogénure de stearimonium, de l'halogénure de behentrimonium, de l'halogénure de dodécyltriméthylammonium, de l'halogénure de didodécyldiméthylammonium, de l'halogénure de tétradécyltriméthylammonium, de l'halogénure de distéaryldiméthylammonium, de l'halogénure de dicétyldiméthylammonium, de la béhénamidopropyldiméthylamine, de la béhénamidopropyldiéthylamine, de la béhénamidoéthyldiéthylamine, de la béhénamidoéthyldiméthylamine, de l'arachidamidopropyldiméthylamine, de l'arachidamidopropyldiéthylamine, de l'arachidamidoethyidiéthylamine et de l'arachidamidoethyidiméthylamine.

14. Composition de conditionnement capillaire selon l'une quelconque des revendications 1 à 13, dans laquelle l'au moins un composant (B2) est choisi dans le groupe constitué des alcools gras, des acides gras, des dérivés d'alcools gras et des dérivés d'acides gras.

15. Composition de conditionnement capillaire selon l'une quelconque des revendications 1 à 14, dans laquelle le rapport pondéral de l'au moins un composant (B2) à l'au moins un composant (B1) est dans la plage de ≥ 1,0:1,0 à ≤ 5,0:1,0.

16. Composition de conditionnement capillaire selon l'une quelconque des revendications 1 à 15, dans laquelle la composition de conditionnement capillaire est exempte de polysaccharides cationiques.

17. Procédé de réduction du volume des cheveux, comprenant les étapes de
a) traitement des cheveux avec une composition de conditionnement capillaire selon l'une quelconque des revendications 1 à 16, et
b) maintien de la composition de conditionnement capillaire en contact avec les cheveux pendant une durée dans la plage de ≥ 1 à ≤ 60 minutes, avant que les cheveux soient séchés.

18. Utilisation d'une composition de conditionnement capillaire selon l'une quelconque des revendications 1 à 16 pour la réduction du volume des cheveux.
